# EUROPÄISCHE PATENTANMELDUNG

(11) **EP 4 410 416 A1**
(43) Veröffentlichungstag der Anmeldung: **07.08.2024**
(21) Anmeldenummer: 24152247.3
(22) Anmeldetag: 16.01.2024
(51) Int. Cl.: B01F 33/453, B01F 35/513, B01F 101/22, B01F 101/44

(54) **MISCHVORRICHTUNG SOWIE EINMALVORRICHTUNG FÜR EINE MISCHVORRICHTUNG**

(30) Priorität: 31.01.2023 EP 23154333
(71) Anmelder: Levitronix GmbH, 8048 Zürich (CH)
(72) Erfinder: Holenstein, Thomas, Dr., 5222 Umiken (CH); Schlegel, Nils, 5417 Untersiggenthal (CH); Wassmer, Florian, 5000 Aarau (CH)
(74) Vertreter: IPS Irsch AG

(57) **Zusammenfassung**

Es wird eine Mischvorrichtung zum Mischen von mindestens zwei Substanzen vorgeschlagen mit einer Einmalvorrichtung (100), die für den Einmalgebrauch ausgestaltet ist, und mit einer wiederverwendbaren Vorrichtung (200), die für den Mehrfachgebrauch ausgestaltet ist, wobei die Einmalvorrichtung (100) ein Flügelrad (10) mit einem Rotorgehäuse (2) und mit mehreren Flügeln (7) zum Mischen der Substanzen, sowie einen Becher (81) zur Aufnahme des Rotorgehäuses (2) umfasst, wobei das Flügelrad (10) zum Rotieren um eine Solldrehachse (D) ausgestaltet ist, welche eine axiale Richtung (A) definiert, wobei das Flügelrad (10) einen Aussendurchmesser (DA) aufweist, welcher die maximale Erstreckung des Flügelrads (10) in einer radialen Richtung senkrecht zur axialen Richtung (A) ist, wobei in dem Rotorgehäuse (2) ein magnetisch wirksamer Kern (3) vorgesehen ist, wobei der Becher (81) formstabil ausgestaltet ist, wobei die wiederverwendbare Vorrichtung (200) einen Stator (4) umfasst, mit welchem das Flügelrad (10) im Betriebszustand berührungslos magnetisch zur Rotation um die Solldrehachse (D) antreibbar und magnetisch bezüglich des Stators (4) lagerbar ist, wobei der Stator (4) mehrere Wicklungen (6) zur Erzeugung eines elektromagnetischen Drehfelds zum Antreiben und Lagern des Flügelrads (10) umfasst, und wobei der Stator ein Statorgehäuse (5) mit einer Ausnehmung (51) aufweist, welche zur Aufnahme des Bechers (81) mit dem darin angeordneten Rotorgehäuse (2) ausgestaltet ist, sodass der Becher (81) in die Ausnehmung einsetzbar ist. An dem Flügelrad (10) sind mehrere Stabilisierungselemente (9) zur Stabilisierung des Flügelrads (10) gegen Verkippungen vorgesehen, wobei jedes Stabilisierungselement (9) zwischen zwei Flügeln (7) angeordnet ist, und sich bezüglich der radialen Richtung von einer radialen Innenkante (91) bis zu einer radialen Aussenkante (92) erstreckt, und wobei jede radiale Aussenkante (92) einen Abstand (D92) von der Solldrehachse (D) hat, der kleiner ist als der halbe Aussendurchmesser (DA) des Flügelrads (7). Ferner wird eine Einmalvorrichtung (100) für eine solche Mischvorrichtung vorgeschlagen.

## Beschreibung

Die Erfindung betrifft eine Mischvorrichtung zum Mischen von mindestens zwei Substanzen sowie eine Einmalvorrichtung für eine Mischvorrichtung gemäss dem Oberbegriff des unabhängigen Patentanspruchs der jeweiligen Kategorie.

Mischvorrichtungen zum Mischen von mindestens zwei Substanzen, beispielsweise von zwei Flüssigkeiten oder von einer Flüssigkeit mit einem Pulver oder von Flüssigkeiten oder Suspensionen mit Gasen werden in vielen technischen Gebieten eingesetzt. Bei vielen Anwendungen kommt dabei der Reinheit des Mischbehälters, in welchem die Vermischung stattfindet, sowie der darin befindlichen Komponenten eine sehr grosse Bedeutung zu. Als Beispiele seien hier die Pharmaindustrie oder die biotechnologische Industrie genannt. Hier werden häufig Lösungen und Suspensionen hergestellt, die eine sorgfältige Durchmischung der Substanzen verlangen.

In der Pharmaindustrie müssen beispielsweise bei der Herstellung von pharmazeutisch wirksamen Substanzen höchste Ansprüche an die Reinheit gestellt werden, oft müssen die mit den Substanzen in Kontakt kommenden Komponenten sogar steril sein. Ähnliche Anforderungen ergeben sich auch in der Biotechnologie, beispielsweise bei der Herstellung, Behandlung oder Züchtung von biologischen Substanzen, Zellen oder Mikroorganismen, wo ein extrem hohes Mass an Reinheit gewährleistet sein muss, um die Brauchbarkeit des hergestellten Produkts nicht zu gefährden.

Bioreaktoren werden beispielsweise zur Gewinnung von Substanzen z.B. Proteinen, oder zur Züchtung von Zellen oder anderem biologischem Material eingesetzt. Dabei können Bioreaktoren sowohl in kontinuierlichen Prozessen als auch in Batch Prozessen betrieben werden. Bei diesen Verfahren ist es eine übliche Methode, das Fluid, beispielsweise eine Zellbrühe (cell broth), dem Bioreaktor zu entnehmen, einer Filtervorrichtung zuzuführen, und das Retentat wieder in den Bioreaktor zurückzuführen. Die zu extrahierende Substanz wird dann als Filtrat bzw. als Permeat der Filtervorrichtung entnommen und abgeführt.

In Bioreaktoren benötigt man daher Mischvorrichtungen, um beispielsweise eine kontinuierliche Durchmischung der Zellbrühe beziehungsweise deren kontinuierliche Zirkulation im Mischbehälter zu gewährleisten. Dabei muss eine sehr hohe Reinheit gewährleistet sein, um die Substanzen oder die erzeugten Produkte vor Kontaminationen zu schützen.

Daher ist die Sterilität in derartigen Prozessen, in denen beispielsweise biologische Aktivitäten stattfinden, von sehr grosser Bedeutung. Das Sterilisieren der Vorrichtungen, beispielsweise mittels Dampfsterilisation, stellt sehr häufig einen zeit- und kostenintensiven Faktor dar. Deshalb besteht heute die zunehmende Tendenz, für solche biotechnologischen Prozesse Komponenten der jeweiligen Vorrichtung als Einmalteile auszugestalten, um aufwändige Sterilisationsprozesse zu vermeiden oder auf ein Minimum zu reduzieren. Insbesondere werden diejenigen Komponenten, die während des Prozesses mit den biologischen Substanzen in direkten Kontakt kommen, häufig als Einmalteile ausgestaltet. Der Begriff Einmalteile (single use) bezeichnet dabei Teile bzw. Komponenten, die bestimmungsgemäss nur einmal benutzt werden dürfen. Nach der Anwendung werden die Einmalteile entsorgt und für die nächste Anwendung durch neue, das heisst noch nicht gebrauchte Einmalteile, ersetzt.

Die Einmalteile werden vor ihrer Verwendung sterilisiert, beispielsweise durch Beaufschlagung mit Gamma-Strahlung. Diese Einmalteile oder Einmalvorrichtungen müssen dann mit anderen Komponenten beispielsweise einer für den Mehrfachgebrauch ausgestalteten wiederverwendbaren Vorrichtung zusammengesetzt werden. Bei der Herstellung bzw. der Konzipierung von Einmalvorrichtungen für den Einmalgebrauch ist es ein wichtiges Kriterium, dass sie in möglichst einfacher Weise mit der wiederverwendbaren Vorrichtung bzw. deren Komponenten zusammengesetzt werden können. Es ist wünschenswert, dass dieses Zusammensetzen mit möglichst geringem Aufwand, mit wenigen Handgriffen, rasch und vorzugsweise ohne Werkzeug erfolgen kann. Daher ist man bestrebt, die Einmalvorrichtungen und die wiederverwendbaren Vorrichtungen so auszugestalten, dass sie in möglichst einfacher Weise zusammengefügt bzw. voneinander getrennt werden können.

Um die Reinheitserfordernisse für den Prozess möglichst gut erfüllen zu können, ist man bemüht, die Anzahl der mit den jeweiligen Substanzen in Kontakt kommenden Komponenten einer Mischvorrichtung möglichst klein zu halten. Hierzu sind elektromagnetisch betriebene Mischvorrichtungen bekannt, bei denen ein Rotor, der üblicherweise ein Flügelrad umfasst oder antreibt, in dem Mischbehälter angeordnet ist. Ausserhalb des Mischbehälters ist dann ein Stator vorgesehen, welcher mittels magnetischer bzw. elektromagnetischer Felder den Rotor durch die Wand des Mischbehälters berührungslos antreibt und berührungslos magnetisch in einer Sollposition lagert. Dieses "berührungslose" Konzept hat insbesondere auch den Vorteil, dass keine mechanischen Lager oder Durchführungen in den Mischbehälter benötigt werden, die eine Ursache von Verunreinigungen bzw. Kontaminationen bilden können.

Eine besonders effiziente derartige Vorrichtung, mit welcher Substanzen in einem Bioreaktor zirkuliert bzw. durchmischt werden, wird im Rahmen der EP-B-2 065 085 offenbart. Hier bilden der Stator und der in dem Mischbehälter angeordnete Rotor einen lagerlosen Motor. Mit dem Begriff lagerloser Motor ist dabei ein elektromagnetischer Drehantrieb gemeint, bei welchem der Rotor vollkommen magnetisch bezüglich des Stators gelagert ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den Wicklungen des Stators lässt sich ein magnetisches Drehfeld erzeugen, welches zum einen ein Drehmoment auf den Rotor ausübt, das dessen Rotation bewirkt, und welches zum anderen eine beliebig einstellbare Querkraft auf den Rotor ausübt, sodass dessen radiale Position aktiv steuerbar bzw. regelbar ist.

Bei Mischvorrichtungen mit magnetisch gelagertem Rotor bzw. magnetisch gelagertem Flügelrad können sich daraus Probleme ergeben, dass die magnetische Lagerung nicht beliebig hoch belastbar ist. Dies trifft insbesondere auch bei solchen Ausgestaltungen zu, bei welchem mindestens ein Freiheitsgrad des Rotors nur passiv magnetisch durch Reluktanzkräfte stabilisiert ist, also nicht aktiv ansteuerbar oder regelbar ist. Werden die diesen Freiheitsgrad betreffenden Kräfte oder Momente auf das Flügelrad zu gross, so ist eine zuverlässige magnetische Lagerung des Rotors nicht mehr gewährleistet. Als Beispiel seien hier Verkippungen des Rotors bzw. des Flügelrads bezüglich der durch die Solldrehachse festgelegten axialen Richtung genannt. Werden die auf das Flügelrad im Betriebszustand einwirkenden Kippmomente zu gross, so reichen die den Rotor stabilisierenden Reluktanzkräfte nicht mehr aus, um genügend grosse Rückstellmomente zu erzeugen, sodass es beispielsweise zu einem Anschlagen des Flügelrads an die das Flügelrad umgebende Wandung(en) kommen kann.

Ausgehend von diesem Stand der Technik ist es daher eine Aufgabe der Erfindung, eine Mischvorrichtung mit einem magnetisch gelagerten Flügelrad vorzuschlagen, welche eine Einmalvorrichtung für den Einmalgebrauch und eine wiederverwendbare Vorrichtung für den Mehrfachgebrauch umfasst, wobei das Flügelrad besser gegen zu starke Verkippungen gesichert ist. Ferner ist es eine Aufgabe der Erfindung, eine Einmalvorrichtung für eine solche Mischvorrichtung vorzuschlagen.

Die diese Aufgabe lösenden Gegenstände der Erfindung sind durch die Merkmale des unabhängigen Patentanspruchs der jeweiligen Kategorie gekennzeichnet.

Erfindungsgemäss wird also eine Mischvorrichtung zum Mischen von mindestens zwei Substanzen vorgeschlagen mit einer Einmalvorrichtung, die für den Einmalgebrauch ausgestaltet ist, und mit einer wiederverwendbaren Vorrichtung, die für den Mehrfachgebrauch ausgestaltet ist, wobei die Einmalvorrichtung ein Flügelrad mit einem Rotorgehäuse und mit mehreren Flügeln zum Mischen der Substanzen, sowie einen Becher zur Aufnahme des Rotorgehäuses umfasst, wobei das Flügelrad zum Rotieren um eine Solldrehachse ausgestaltet ist, welche eine axiale Richtung definiert, wobei das Flügelrad einen Aussendurchmesser aufweist, welcher die maximale Erstreckung des Flügelrads in einer radialen Richtung senkrecht zur axialen Richtung ist, wobei in dem Rotorgehäuse ein magnetisch wirksamer Kern vorgesehen ist, wobei der Becher formstabil ausgestaltet ist, wobei die wiederverwendbare Vorrichtung einen Stator umfasst, mit welchem das Flügelrad im Betriebszustand berührungslos magnetisch zur Rotation um die Solldrehachse antreibbar und magnetisch bezüglich des Stators lagerbar ist, wobei der Stator mehrere Wicklungen zur Erzeugung eines elektromagnetischen Drehfelds zum Antreiben und Lagern des Flügelrads umfasst, und wobei der Stator ein Statorgehäuse mit einer Ausnehmung aufweist, welche zur Aufnahme des Bechers mit dem darin angeordneten Rotorgehäuse ausgestaltet ist, sodass der Becher in die Ausnehmung einsetzbar ist. An dem Flügelrad sind mehrere Stabilisierungselemente zur Stabilisierung des Flügelrads gegen Verkippungen vorgesehen, wobei jedes Stabilisierungselement zwischen zwei Flügeln angeordnet ist, und sich bezüglich der radialen Richtung von einer radialen Innenkante bis zu einer radialen Aussenkante erstreckt, und wobei jede radiale Aussenkante einen Abstand von der Solldrehachse hat, der kleiner ist als der halbe Aussendurchmesser des Flügelrads.

Es hat sich gezeigt, dass durch diese Stabilisierungselemente am Flügelrad, die an den Flügeln und/oder am Rotorgehäuse angeordnet sind, eine deutlich bessere Stabilisierung des rotierenden Flügelrads gegen Verkippungen relativ zur axialen Richtung resultiert. Ferner ist auch die axiale Position des Flügelrads besser stabilisiert, sodass das Flügelrad besser in seiner Sollposition bezüglich der axialen Richtung bleibt und sich weniger leicht in axialer Richtung verschiebt. Wenn die Mischvorrichtung nach dem Prinzip des lagerlosen Motors ausgestaltet ist, ist das Flügelrad üblicherweise bezüglich dieser drei Freiheitsgrade, nämlich die beiden Freiheitsgrade der Verkippung und der Freiheitsgrad der axialen Position, passiv magnetisch, also nicht ansteuerbar, stabilisiert bzw. gelagert. Durch die Stabilisierungselemente wird dann die magnetische Lagerung gerade bezüglich der Freiheitsgrade entlastet, die passiv magnetisch stabilisiert sind. Hieraus resultiert eine deutlich stabilere magnetische Lagerung des Flügelrads. Durch die erhöhte Stabilität kann dann das Flügelrad auch mechanisch stärker belastet werden, beispielsweise können längere oder breitere Flügel verwendet werde, was sich positiv auf die Effizienz der Mischvorrichtung auswirkt.

Insbesondere hat es sich gezeigt, dass die zusätzliche Stabilisierung durch die Stabilisierungselemente bei weitem eine mögliche Destabilisierung überwiegt, welche aus Turbulenzen resultieren kann, die von den Stabilisierungselementen generiert werden.

Es sind Ausgestaltungen möglich, bei denen jede radiale Aussenkante einen Abstand von der Solldrehachse hat, der höchstens 90% oder höchstens 80% oder höchstens 70% des halben Aussendurchmessers des Flügelrads beträgt.

Vorzugsweise ist jeder Flügel bezüglich der radialen Richtung mit einem Radialabstand von der Solldrehachse angeordnet, wobei der Radialabstand von null verschieden ist, und wobei die radiale Innenkante jedes Stabilisierungselements einen Abstand von der Solldrehachse hat, der grösser ist als der Radialabstand. Das bedeutet, dass sich die Flügel bezüglich der radialen Richtung näher an die Solldrehachse erstrecken als die radialen Innenkannten der Stabilisierungselemente, oder umgekehrt ausgedrückt, bezüglich der radialen Richtung liegen die radialen Innenkanten der Stabilisierungselemente weiter aussen als der innenliegende Beginn der Flügel, d.h. die Stabilisierungselemente beginnen weiter aussen als die Flügel.

Gemäss einer bevorzugten Ausführungsform weist jeder Flügel eine Vorderseite, eine Rückseite, eine Oberkante und eine Unterkante auf, wobei die Vorderseite beim Rotieren des Flügelrads der Rückseite vorausläuft, wobei die Unterkante und die Oberkante den Flügel bezüglich der axialen Richtung begrenzen, wobei die Oberkante an dem Ende des Flügels angeordnet ist, welches dem Rotorgehäuse abgewandt ist, und wobei die Stabilisierungselemente auf den Vorderseiten und/oder auf den Rückseiten der Flügel angeordnet sind.

Bei dieser Ausführungsform sind die Stabilisierungselemente also an den Vorderseiten oder an den Rückseiten der Flügel angeordnet und somit auf den Flächen der Flügel, die primär für die Durchmischung der Substanzen sorgen. Da jedes Stabilisierungselement auf der Vorderseite oder auf der Rückseite eines Flügels angeordnet ist, ist das Stabilisierungselement in Umfangsrichtung gesehen zwischen diesem Flügel und seinem in Umfangsrichtung benachbarten Flügel angeordnet.

Vorzugsweise sind die Stabilisierungselement jeweils zumindest im Wesentlichen rechtwinklig zu der jeweiligen Vorderseite oder Rückseite des Flügels angeordnet, sodass sich die Stabilisierungselemente jeweils von der Vorderseite bzw. von der Rückseite in Umfangsrichtung erstrecken und mit der Vorderseite bzw. mit der Rückseite einen Winkel von zumindest näherungsweise 90° einschliessen. Es ist aber auch möglich, dass die Stabilisierungselemente schräg zu der Vorderseite bzw. zu der Rückseite angeordnet sind, also mit der Vorderseite bzw. mit der Rückseite einen stumpfen Winkel oder einen spitzen Winkel einschliessen.

Bevorzugt sind also solche Ausgestaltungen, bei denen die Stabilisierungselemente mit der Vorderseite oder mit der Rückseite jeweils einen von 0° und von 180° verschiedenen Winkel einschliessen, der besonders bevorzugt im Bereich von 40° bis 140° liegt.

Eine weitere vorteilhafte Massnahme besteht darin, dass die Stabilisierungselemente symmetrisch angeordnet sind, derart, dass jeder Flügel, an dessen Vorderseite ein Stabilisierungselement vorgesehen ist, auch auf der Rückseite mit einem Stabilisierungselement versehen ist. Diese bezüglich des jeweiligen Flügels symmetrische Ausgestaltung resultiert in eine austarierte Axialposition des Flügelrads. Insbesondere können grössere Auslenkungen des Flügelrads, beispielsweise durch zusätzliche Kippmomente, vermieden werden.

Ferner hat es sich als vorteilhaft erwiesen, wenn die Stabilisierungselemente näher an der Unterkante als an der Oberkante des jeweiligen Flügels angeordnet sind. Die Stabilisierungselemente sind dann bezüglich der axialen Richtung näher am magnetisch wirksamen Kern angeordnet, was die Stabilisierung des Flügelrads positiv beeinflusst. Auch hat es sich im Hinblick auf die erzielte Stabilisierung als vorteilhaft erwiesen, die Stabilisierungselemente bezüglich ihrer radialen Position näher an die Solldrehachse anzuordnen, sodass die jeweilige radiale Innenkante des Stabilisierungselements näher am radial innenliegenden Ende des Flügels angeordnet ist als am radial aussenliegenden Ende des Flügels.

Insgesamt ist es also vorteilhaft, wenn die Stabilisierungselemente sowohl bezüglich der axialen Richtung als auch bezüglich der radialen Richtung nahe am Zentrum des magnetisch wirksamen Kerns angeordnet werden.

Gemäss einer bevorzugten Ausführungsform ist jeder Flügel gekrümmt ausgestaltet, sodass die Vorderseite oder die Rückseite konkav ausgestaltet sind.

Eine weitere bevorzugte Ausführungsform besteht darin, dass die Stabilisierungselemente Ringsegmente umfassen, die sich jeweils von der Vorderseite eines Flügels bis zu der Rückseite eines benachbarten Flügels erstrecken. Bei dieser Ausführungsform wird jeweils die Vorderseite eines Flügels durch ein Ringsegment mit der Rückseite des benachbarten Flügels verbunden.

Gemäss einer bevorzugten Ausgestaltung ist das Rotorgehäuse zylindrisch mit einem äusseren Gehäusedurchmesser ausgestaltet, sowie mit einer oberen Stirnfläche und mit einer unteren Stirnfläche, welche das Rotorgehäuse bezüglich der axialen Richtung begrenzen, wobei optional auf der oberen Stirnfläche des Rotorgehäuses eine Nabenscheibe angeordnet ist, auf welcher die Flügel vorgesehen sind. Es ist also möglich, das Flügelrad mit einer Nabenscheibe auszugestalten, auf welcher die Flügel angeordnet sind, und diese Nabenscheibe dann mit dem Rotorgehäuse zu verbinden, beispielsweise durch eine Rastverbindung, eine Bajonettverbindung oder eine Schnappverbindung. Andererseits ist es auch möglich, dass die Flügel direkt auf oder in einer der Stirnflächen des Rotorgehäuses angeordnet sind.

Gemäss einer bevorzugten Ausführungsform sind die Stabilisierungselemente auf der oberen Stirnfläche des Rotorgehäuses oder auf der Nabenscheibe angeordnet, wobei der Abstand jeder radialen Aussenkante von der Solldrehachse grösser ist als der halbe Gehäusedurchmesser des Rotorgehäuses. Dadurch ragen die Stabilisierungselemente bezüglich der radialen Richtung über das Rotorgehäuse hinaus.

Um den für Verkippungen zur Verfügung stehenden Spielraum zu vergrössern, ist es eine bevorzugte Massnahme, dass die obere Stirnfläche und/oder die untere Stirnfläche des Rotorgehäuses mit einer Fase ausgestaltet sind, die sich entlang des gesamten Umfangs der Stirnfläche erstreckt.

Eine weitere vorteilhafte Massnahme besteht darin, dass mindestens eine Entlastungsbohrung vorgesehen ist, die sich in axialer Richtung vollständig durch das Flügelrad hindurch erstreckt.

Ferner wird durch die Erfindung eine Einmalvorrichtung für eine Mischvorrichtung vorgeschlagen, die erfindungsgemäss ausgestaltet ist. Die Einmalvorrichtung umfasst das Flügelrad sowie den Becher zur Aufnahme des Rotorgehäuses.

Gemäss einer bevorzugten Ausführungsform umfasst die Einmalvorrichtung ferner eine Fixiereinrichtung, mit welchem der magnetisch wirksame Kern magnetisch in dem Becher gehalten wird, wobei die Fixiereinrichtung durch Ziehen entfernbar ist. Dadurch kann das Flügelrad, beispielsweise bei der Auslieferung oder dem Versand der Einmalvorrichtung, durch Magnetkräfte in dem Becher fixiert werden. Nach dem Einsetzen der Einmalvorrichtung in die wiederverwendbare Vorrichtung und vor dem Gebrauch wird dann die Fixiereinrichtung durch Ziehen entfernt. Die Fixiereinrichtung umfasst beispielsweise eine metallische Scheibe, die aussen am Boden des Bechers angeordnet ist und mit dem magnetisch wirksamen Kern des Flügelrads zusammenwirkt, sowie ein Band, das an dieser Scheibe befestigt ist, sodass die Scheibe von dem Becher abgezogen werden kann.

Gemäss einer weiteren bevorzugten Ausgestaltung umfasst die Einmalvorrichtung ferner einen flexiblen Mischbehälter für die zu mischenden Substanzen, wobei der Mischbehälter dichtend mit dem Becher zu einer Gesamtheit verbunden ist, und wobei das Flügelrad in der Gesamtheit angeordnet ist. Der Mischbehälter ist beispielsweise als Plastiksack ausgestaltet, der mit dem Becher verschweissbar ist, sodass der Becher mit dem Plastiksack dann die Gesamtheit bildet, in welche die zu mischenden Substanzen eingebracht werden. Der flexible Mischbehälter mit dem daran angeschweissten Becher kann dann in einen formstabilen Stützbehälter eingesetzt werden, wobei der Stator dann den Becher mit dem darin angeordneten Rotorgehäuse aufnimmt.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der Zeichnung zeigen (teilweise im Schnitt):
- Fig. 1:: eine perspektivische Darstellung eines Ausführungsbeispiels einer erfindungsgemässen Mischvorrichtung,
- Fig. 2:: wie Fig. 1, jedoch in einer Explosionsdarstellung,
- Fig. 3:: eine Schnittdarstellung des Ausführungsbeispiels in einem Schnitt entlang der axialen Richtung,
- Fig. 4:: wie Fig. 3, jedoch in einer Explosionsdarstellung,
- Fig. 5:: eine perspektivische Darstellung einer Ausführungsform des Stators einer erfindungsgemässen Mischvorrichtung (ohne Statorgehäuse)
- Fig. 6:: eine schematische Schnittdarstellung des Stators aus Fig. 5 in einem Schnitt entlang der axialen Richtung,
- Fig. 7: eine schematische Schnittdarstellung einer Ausführungsform des Flügelrads in einem Schnitt entlang der axialen Richtung,
- Fig. 8:: eine perspektivische Schnittdarstellung einer Ausgestaltung des Rotorgehäuses mit einer darauf aufgesetzten Nabenscheibe,
- Fig. 9:: eine perspektivische Darstellung eines Ausführungsbeispiels einer Einmalvorrichtung mit einer Fixiereinrichtung,
- Fig. 10:: die Einmalvorrichtung aus Fig. 9 in einem Schnitt entlang der axialen Richtung,
- Fig. 11 - 16:: verschiedene Varianten für die Ausgestaltung des Flügelrads, jeweils in einer perspektivischen Darstellung,
- Fig. 17:: die Variante aus Fig. 16 in einer Seitenansicht,
- Fig. 18 - 23:: weitere Varianten für die Ausgestaltung des Flügelrads, jeweils in einer perspektivischen Darstellung, und
- Fig. 24:: die Variante aus Fig. 23 in einer Aufsicht.

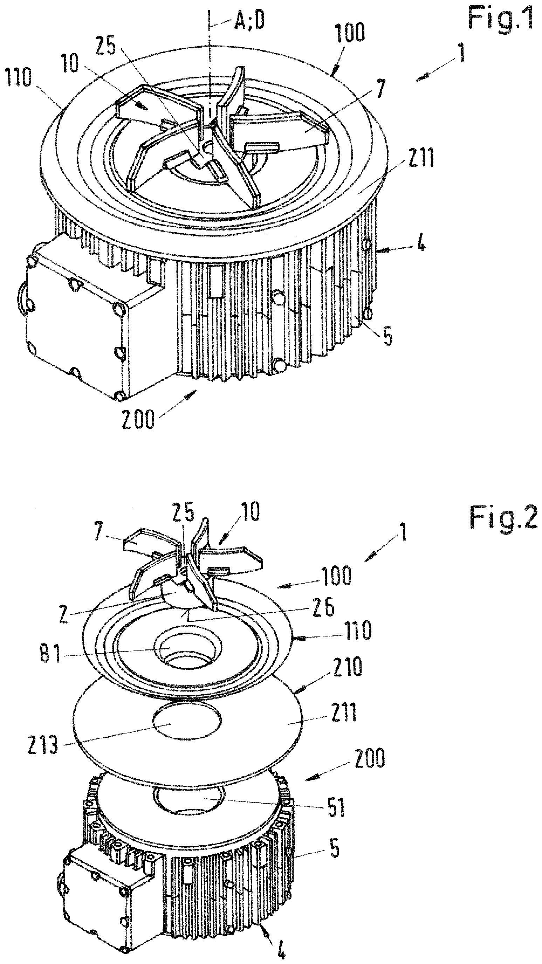

Fig. 1 zeigt in einer perspektivischen Darstellung ein Ausführungsbeispiel einer erfindungsgemässen Mischvorrichtung, die gesamthaft mit dem Bezugszeichen 1 bezeichnet ist. Derartige Mischvorrichtungen 1 können insbesondere in der biotechnologischen Industrie oder in der pharmazeutischen Industrie Verwendung finden. Speziell eignet sich die erfindungsgemässe Mischvorrichtung 1 auch für solche Anwendungen, bei denen ein sehr hohes Mass an Reinheit oder Sterilität derjenigen Komponenten wesentlich ist, die mit den zu mischenden Substanzen in Kontakt kommen. Die erfindungsgemässe Mischvorrichtung 1 kann auch als Bioreaktor oder als Fermenter oder als ein Teil eines Bioreaktors oder Fermenters ausgestaltet sein. Es versteht sich jedoch, dass die Erfindung nicht auf solche Ausgestaltungen beschränkt ist, sondern ganz allgemein Mischvorrichtungen betrifft, mit denen Medien oder Substanzen gemischt werden. Insbesondere können diese Substanzen Fluide oder Feststoffe, vorzugsweise Pulver, sein. Die erfindungsgemässe Mischvorrichtung 1 eignet sich zum Mischen von Flüssigkeiten untereinander und/oder zum Mischen von mindestens einer Flüssigkeit mit einem Pulver oder sonstigen Feststoff und/oder zum Mischen von Gasen mit Flüssigkeiten und/oder Feststoffen.

Um die Reinheit bzw. die Sterilität derjenigen Komponenten zu gewährleisten, die mit den zu mischenden Substanzen in Kontakt kommen, umfasst die erfindungsgemässe Mischvorrichtung 1 eine Einmalvorrichtung, die gesamthaft mit dem Bezugszeichen 100 bezeichnet ist und für den Einmalgebrauch ausgestaltet ist, sowie eine wiederverwendbare Vorrichtung, die gesamthaft mit dem Bezugszeichen 200 bezeichnet ist und die für den dauerhaften Gebrauch, also den Mehrfachgebrauch ausgestaltet ist. Dabei umfasst die Einmalvorrichtung 100 vorzugsweise diejenigen Komponenten, welche während des Mischvorgangs mit den zu mischenden Substanzen in Kontakt kommen.

Mit dem Begriff "Einmalvorrichtung" und anderen Zusammensetzungen mit dem Bestandteil "Einmal", wie z. B. Einmalteil, Einmalkomponente usw., sind dabei solche Vorrichtungen, Komponenten bzw. Teile gemeint, die für den Einmalgebrauch ausgestaltet sind, die also bestimmungsgemäss nur ein einziges Mal benutzt werden können und dann entsorgt werden. Für eine neue Anwendung muss dann ein neues, bisher unbenutztes Einmalteil eingesetzt werden. Bei der Konzipierung bzw. der Ausgestaltung der Einmalvorrichtung 100 sind es daher wesentliche Aspekte, dass die Einmalvorrichtung 100 möglichst einfach und wirtschaftlich herstellbar ist, wenige Kosten verursacht und aus möglichst preisgünstig erhältlichen Materialen herstellbar ist. Ein anderer wesentlicher Aspekt ist es, dass die Einmalvorrichtung 100 in möglichst einfacher Weise mit der wiederverwendbaren Vorrichtung 200 zusammenfügbar ist. Die Einmalvorrichtung 100 soll also in sehr einfacher Weise ersetzt werden können, ohne dass dafür ein hoher Montageaufwand notwendig ist. Besonders bevorzugt soll die Einmalvorrichtung 100 ohne die Verwendung von Werkzeugen mit der wiederverwendbaren Vorrichtung 200 zusammenfügbar sein und von der wiederverwendbaren Vorrichtung 200 trennbar sein.

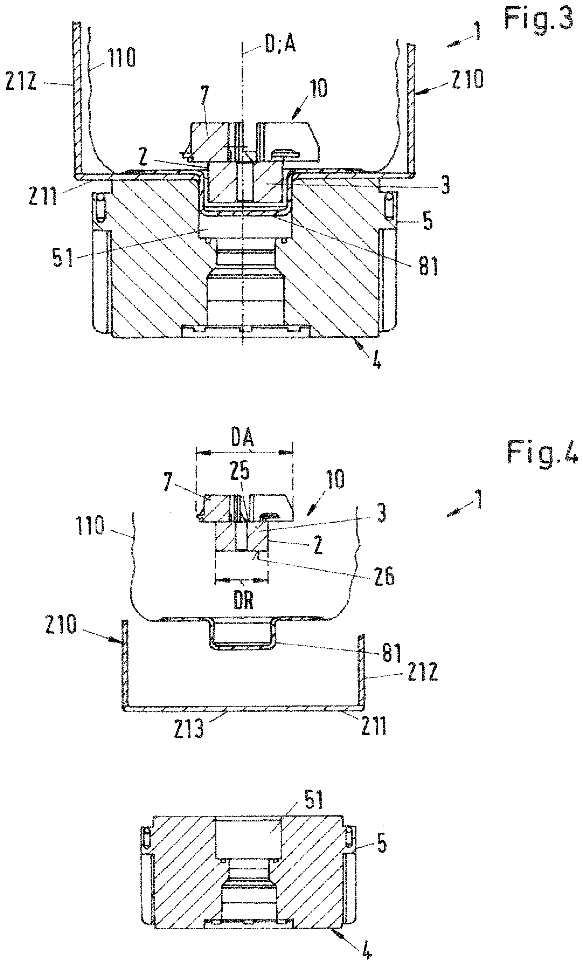

Zum besseren Verständnis zeigt Fig. 2 noch eine perspektivische Explosionsdarstellung des Ausführungsbeispiels der erfindungsgemässen Mischvorrichtung 1 aus Fig. 1. Fig. 3 zeigt eine Schnittdarstellung dieses Ausführungsbeispiels in einem Schnitt entlang einer axialen Richtung A und im assemblierten Zustand. Schliesslich zeigt Fig. 4 das Ausführungsbeispiel in einer zu Fig. 3 analogen Schnittdarstellung, jedoch in einer Explosionsdarstellung.

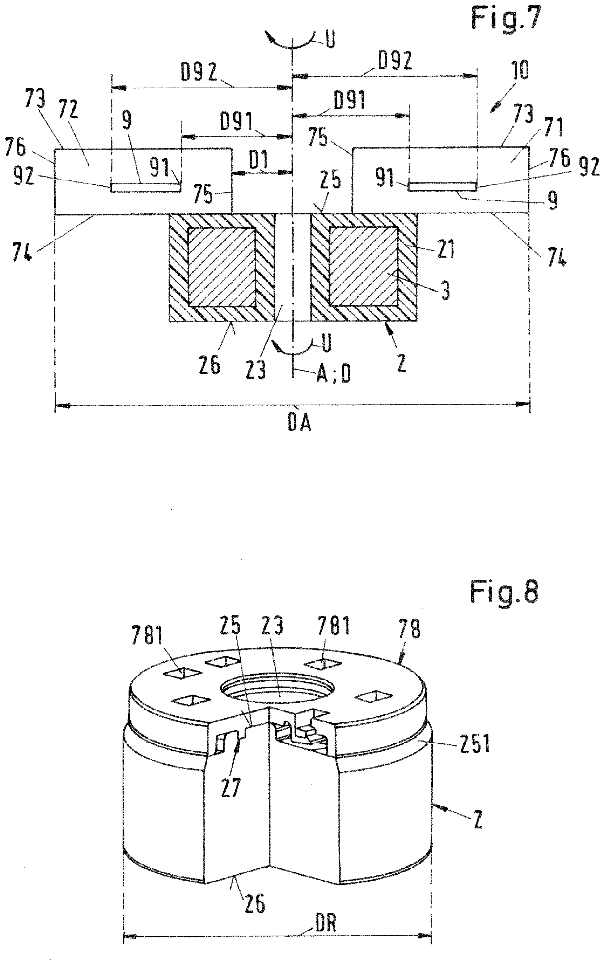

Bei der Mischvorrichtung 1 (Fig. 1 - Fig. 4) umfasst die Einmalvorrichtung 100 mindestens die folgenden Komponenten: ein Flügelrad 10 mit einem Rotorgehäuse 2 und mit mehreren Flügeln 7, hier mit fünf Flügeln 7, zum Mischen der Substanzen, sowie einen Becher 81 zur Aufnahme des Rotorgehäuses 2. Im Rotorgehäuse 2 ist ein magnetisch wirksamer Kern 3 angeordnet, der beispielsweise permanentmagnetisch ausgestaltet ist. Der magnetisch wirksame Kern 3 ist bezüglich des Rotorgehäuses 2 fixiert, derart, dass der magnetisch wirksame Kern 3 sowohl drehfest mit dem Rotorgehäuse 2 verbunden ist und auch keine translatorische Bewegung relativ zum Rotorgehäuse 2 ausführen kann. Dazu ist das Rotorgehäuse 2 beispielsweise mit einem Kunststoff ausgegossen z.B. mit einem Epoxidharz, wodurch der magnetisch wirksame Kern 3 relativ zum Rotorgehäuse 2 fixiert ist. Auch ist es möglich, dass das Rotorgehäuse 2 als Umkapslung 21 des magnetisch wirksamen Kerns 3 ausgestaltet ist (siehe z.B. Fig. 7).

Das Rotorgehäuse 2 ist zylindrisch mit einem äusseren Gehäusedurchmesser DR ausgestaltet. Das Rotorgehäuse 2 hat eine obere Stirnfläche 25 und eine untere Stirnfläche 26, welche das Rotorgehäuse 2 bezüglich der axialen Richtung A begrenzen. Bei dem hier beschriebenen Ausführungsbeispiel sind die fünf Flügel 7 direkt auf der oberen Stirnfläche 25 des Rotorgehäuses 2 angeordnet, wobei die Flügel 7 bezüglich der Umfangsrichtung äquidistant auf der oberen Stirnfläche 25 angeordnet sind. Die Flügel 7 können beispielsweise auf die obere Stirnfläche 25 aufgesetzt und dort befestigt werden, beispielsweise mittels einer Klemm- oder Rastverbindung, oder die Flügel 7 sind mit der oberen Stirnfläche 25 verklebt oder verschweisst. Auch ist es möglich, dass die Flügel 7 integraler Bestandteil des Rotorgehäuses 2 sind.

Gemäss einer ebenfalls bevorzugten Variante (siehe Fig. 8) ist eine separate Nabenscheibe 78 vorgesehen, auf welcher die Flügel 7 angeordnet sind. Diese Nabenscheibe 78 wird dann auf der oberen Stirnfläche 25 des Rotorgehäuses 2 fixiert.

Das Flügelrad 10 mit dem Rotorgehäuse 2 ist zum Rotieren um eine Solldrehachse D ausgestaltet, welche die axiale Richtung A definiert, wobei das Flügelrad 10 berührungslos magnetisch antreibbar und magnetisch lagerbar ist. Dazu ist in dem Rotorgehäuse 2 der magnetisch wirksamen Kern 3 angeordnet. Mit dem "magnetisch wirksamen Kern 3" des Flügelrads 10 ist derjenige Bereich des Flügelrads 10 gemeint, welcher für die Drehmomentbildung sowie für die Erzeugung der magnetischen Lagerkräfte magnetisch mit einem Stator 4 zusammenwirkt.

Das Flügelrad 10 weist einen Aussendurchmesser DA (Fig. 4) auf, womit die maximale Erstreckung des Flügelrads 10 in einer radialen Richtung gemeint ist, wobei die radiale Richtung senkrecht zur axialen Richtung A ist.

Der Becher 81 ist formstabil ausgestaltet und vorzugsweise aus einem Kunststoff gefertigt. Der Becher 81 ist im Wesentlichen zylindrisch und rotationssymmetrisch bezüglich der axialen Richtung A ausgestaltet.

Der Innendurchmesser des Bechers 81 ist so bemessen, dass er geringfügig grösser ist als der Gehäusedurchmesser DR des Rotorgehäuses 2. Geringfügig ist dabei so zu verstehen, dass einerseits das Rotorgehäuse 2 in dem Becher 81 frei rotieren kann, ohne dabei die Innenwand des Bechers 81 zu berühren, und andererseits der Abstand zwischen der zylindrischen Aussenwand des Rotorgehäuses 2 und der Innenwand des Bechers 81 möglichst gering ist, um eine effiziente magnetische Lagerung sowie einen effizienten magnetischen Antrieb des Flügelrads 10 zu gewährleisten.

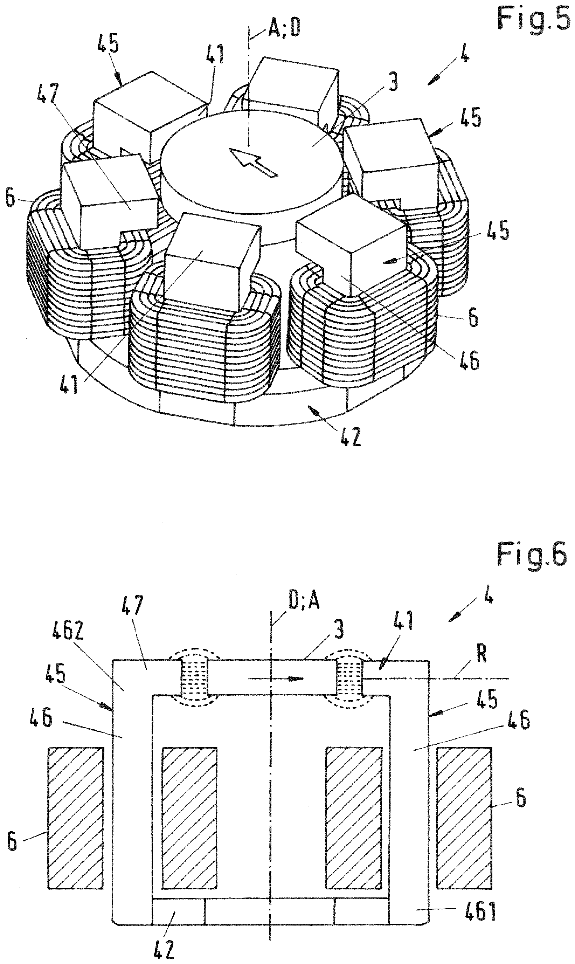

Die wiederverwendbare Vorrichtung 200 umfasst den Stator 4, mit welchem das Flügelrad im Betriebszustand berührungslos magnetisch zur Rotation um die Solldrehachse D antreibbar und magnetisch bezüglich des Stators 4 lagerbar ist, wobei der Stator 4 mehrere Wicklungen 6 (siehe z. B. Fig. 5) zur Erzeugung eines elektromagnetischen Drehfelds zum Antreiben und Lagern des Flügelrads 10 umfasst. Der Stator 4 ist in einem Statorgehäuse 5 angeordnet, welches als Umkapslung des Stators 4 ausgestaltet ist, vorzugsweise als hermetische Umkapslung.

Das Statorgehäuse 5 wird bezüglich der axialen Richtung A von zwei Stirnflächen begrenzt. In einer der beiden Stirnflächen ist eine zentrale Ausnehmung 51 vorgesehen, welche zur Aufnahme des Bechers 81 mit dem darin angeordneten Rotorgehäuse 2 ausgestaltet ist, sodass der Becher 81 in die Ausnehmung 51 einsetzbar ist. Somit kann der im Rotorgehäuse 2 angeordnete magnetisch wirksame Kern 3 magnetisch mit dem Stator 4 wechselwirken und mittels der Wicklungen 6 des Stators 4 berührungslos zur Rotation angetrieben werden. Die Ausnehmung 51 im Statorgehäuse 5 ist bezüglich ihrer radialen Abmessung so ausgestaltet, dass einerseits der Becher 81 in einfacher Weise in die Ausnehmung 51 eingesetzt werden kann, und dass andererseits der in radialer Richtung gemessene Abstand zwischen dem Statorgehäuse 5 und dem magnetisch wirksamen Kern 3 möglichst gering ist. Die Abmessungen des Bechers 81 und der Ausnehmung 51 sind vorzugsweise so aufeinander abgestimmt, dass die Ausnehmung 51 den Becher 81 im zusammengesetzten Zustand eng umschliesst und mit ihrer Wandung an der Mantelfläche des Bechers 81 anliegt.

Vorzugsweise umfasst die Einmalvorrichtung 100 ferner einen flexiblen Mischbehälter 110 zur Aufnahme der zu mischenden Substanzen, welcher aus einem Kunststoff hergestellt ist. Der flexible Mischbehälter 110 ist in Fig. 1 und in Fig. 2 lediglich mit seinem darstellungsgemäss unteren Rand angedeutet. In den Fig. 3 und Fig. 4 ist etwas mehr von dem Mischbehälter 110dargestellt. Der Mischbehälter 110 ist vorzugsweise ein flexibler Beutel, beispielsweise ein Kunststoffsack, der zusammengefaltet werden kann, sodass er bei der Lagerung möglichst wenig Platz beansprucht. Der flexible Mischbehälter 110 ist dichtend mit dem Becher 81 zu einer Gesamtheit verbunden, in welcher das Mischen der Substanzen stattfindet. Der flexible Mischbehälter 110 ist beispielsweise mit dem Becher 81 verschweisst oder verklebt.

Der Mischbehälter 110 kann in an sich bekannter Weise mindestens einen Einlass (nicht dargestellt) aufweisen, durch welchen die zu mischenden Substanzen in den Mischbehälter 110 einführbar sind. Ferner können weitere Einlässe vorgesehen sein, beispielsweise für die Zuführung weiterer Substanzen, für die Aufnahme von Sonden oder Messsensoren, mit denen Parameter während des Mischprozesses überwacht werden, z. B. Temperatur, Druck, Konzentrationen etc. Auch können die Einlässe für den Stoffaustausch insbesondere während des Mischprozesses genutzt werden. Speziell können hierbei, beispielsweise bei einer Ausgestaltung als Bioreaktor, notwendige Gase zu- oder abgeführt werden. Insbesondere bei der Zucht von Mikroorganismen oder biologischem Gewebe oder Zellen ist es häufig eine Notwendigkeit, dass dem Mischbehälter 110 Sauerstoff bzw. Luft zugeführt und andere Gase, insbesondere Kohlendioxid, aus dem Mischbehälter 110 abgeführt werden können. Ferner umfasst der Mischbehälter 110 mindestens ein Auslass (nicht dargestellt), durch welchen Substanzen, beispielsweise die durchmischten Substanzen abführbar sind.

Da der flexible Mischbehälter 110 häufig an die spezifische Anwendung angepasst ist, beispielsweise bezüglich der Anzahl und der Anordnung der Einlässe, ist es eine bevorzugte Variante, dass die Einmalvorrichtung 100 ohne den flexiblen Mischbehälter 110 verpackt und an den Anwender ausgeliefert wird. Der Anwender stellt dann den für die spezifische Anwendung geeigneten flexiblen Mischbehälter 110 zur Verfügung und verbindet diesen dichtend mit dem Becher 81 der Einmalvorrichtung 100 zu der Gesamtheit für die Aufnahme der zu mischenden Substanzen.

Für die Anwendung wird der flexible Mischbehälter 110 mit der daran befestigten Becher 81 in einen formstabilen Stützbehälter 210 eingesetzt, der beispielsweise Bestandteil der wiederverwendbaren Vorrichtung 200 ist. Der Stützbehälter 210 ist im Wesentlichen zylindrisch ausgestaltet mit einer kreisförmigen Bodenplatte 211 und einer zylindrischen Seitenwand 212. Die Seitenwand 212 ist in Fig. 3 und in Fig. 4 angedeutet. In den Fig. 1 und Fig. 2 ist aus Gründen der besseren Übersicht nur die Bodenplatte 211 dargestellt.

An seiner Oberseite ist der im Wesentlichen zylindrisch ausgestaltete Stützbehälter 210 offen, sodass der Mischbehälter 110 problemlos in den Stützbehälter 210 eingebracht werden kann. Der Stützbehälter 210 ist beispielsweise als Tank ausgestaltet und z.B. aus Stahl, insbesondere einem Edelstahl gefertigt.

In der Bodenplatte 211 des Stützbehälters 210 ist eine zentral angeordnete und kreisförmig ausgestaltete Öffnung 213 vorgesehen, welche so bemessen ist, dass der Becher 81 durch diese Öffnung 213 hindurchgeführt werden kann und an seinem Rand durch die Bodenplatte 211 gestützt wird.

Das Statorgehäuse 5 ist so an der Aussenseite der Bodenplatte 211 angeordnet, dass die zentrale Ausnehmung 51 im Statorgehäuse 5 mit der Öffnung 213 in der Bodenplatte 211 fluchtet, sodass der Becher 81 durch die Öffnung 213 in der Bodenplatte 211 hindurch in die Ausnehmung 51 des Statorgehäuses 5 einsetzbar ist. Vorzugsweise ist das Statorgehäuse 5 an der Aussenseite der Bodenplatte 211 befestigt, beispielsweise mittels Schrauben (nicht dargestellt).

Das Zusammensetzen der Einmalvorrichtung 100 und der wiederverwendbaren Vorrichtung 200 zu der Mischvorrichtung 1 ist äusserst einfach, sowie schnell und insbesondere ohne Werkzeuge durchführbar. Dazu wird der üblicherweise für die Lagerung zusammengefaltete Mischbehälter 110 mit der daran dichtend befestigten Becher 81 in den Stützbehälter 210 eingelegt, und der Becher 81 mit dem darin angeordneten Flügelrad 10 durch die Öffnung 213 in der Bodenplatte 211 in die Ausnehmung 51 des Statorgehäuses 5 eingesetzt. Schon dann ist die Mischvorrichtung 1 bereit für die Anwendung. Nach der Anwendung wird der Mischbehälter 110 mitsamt dem Becher 81 und dem Flügelrad 10 einfach aus dem Stützbehälter 210 herausgezogen. Diese besonders einfache und problemlose Verbindung bzw. Trennung der Einmalvorrichtung 100 mit bzw. von der wiederverwendbaren Vorrichtung 200 trägt somit einem wesentlichen Aspekt der Ausgestaltung für den Einmalgebrauch Rechnung.

Es sind auch solche Ausgestaltungen möglich, bei denen der Mischbehälter 110 als formstabiler Mischbehälter 110 ausgestaltet ist, sodass es kein Stützbehälter 210 benötigt wird. Ist der Mischbehälter 110 als formstabiler Mischbehälter 110 ausgestaltet, so kann er für den Einmalgebrauch ausgestaltet sein und beispielsweise aus einem Kunststoff gefertigt sein, oder der Mischbehälter 110 ist für einen mehrfachen Gebrauch, also wiederverwendbar, ausgestaltet und beispielsweise aus einem metallischen Material wie Edelstahl gefertigt.

Falls der Mischbehälter 110 als formstabiler Mischbehälter 110 ausgestaltet ist, so kann er auch einstückig mit dem Becher 81 ausgestaltet sein, sodass der Becher 81 integraler Bestandteil des Mischbehälters ist. Es sind natürlich auch solche Ausgestaltungen möglich, bei welchen der Becher 81 eine separate Komponente ist, die dichtend mit dem formstabilen Mischbehälter 110 verbindbar ist.

Im Folgenden wird anhand der Fig. 5 und Fig. 6 eine bevorzugte Ausführungsform des Stators 4 der erfindungsgemässe Mischvorrichtung 1 erläutert. Fig. 5 zeigt diese Ausführungsform des Stators 4 in einer perspektivischen Darstellung. Fig. 6 zeigt den Stator 4 in einem Schnitt entlang der axialen Richtung A. Zum besseren Verständnis ist in den Fig. 5 und Fig. 6 das Statorgehäuse 5 nicht dargestellt. Ferner ist zum besseren Verständnis in den Fig. 5 und Fig. 6 von dem Flügelrad 10 jeweils der magnetisch wirksame Kern 3 dargestellt, welcher mit dem Stator 4 als elektromagnetischer Drehantrieb zusammenwirkt.

Dieser elektromagnetische Drehantrieb, der den Stator 4 und den magnetisch wirksamen Kern 3 umfasst, ist hier als Tempelmotor ausgestaltet.

Bei einem Tempelmotor umfasst der Stator 4 eine Mehrzahl von Spulenkernen 45, hier sechs Spulenkerne 45, von denen jeder einen stabförmigen Längsschenkel 46 umfasst, welcher sich von einem ersten Ende 461 in axialer Richtung A bis zu einem zweiten Ende 462 erstreckt, sowie einen Querschenkel 47, welcher an dem zweiten Ende 462 des Längsschenkels 46 angeordnet ist. Jeder Querschenkel 47 erstreckt sich in radialer Richtung auf den magnetisch wirksamen Kern 3 zu und wird durch eine dem magnetisch wirksamen Kern 3 zugewandte Stirnfläche begrenzt.

Die ersten Enden 461 aller Längsschenkel 46 sind über einen Rückschluss 42 magnetisch leitend miteinander verbunden. Der Rückschluss 42 ist vorzugsweise ringförmig ausgestaltet oder umfasst mehrere Segmente, welche die Längsschenkel 46 miteinander verbinden. Sowohl der Rückschluss 42 als auch die Spulenkerne 45 des Stators 4 sind jeweils aus einem weichmagnetischen Material gefertigt, weil sie als Flussleitelemente zur Führung des magnetischen Flusses dienen. Geeignete weichmagnetische Materialien für die Spulenkerne 45 und den Rückschluss 42 sind beispielsweise ferromagnetische oder ferrimagnetische Materialien, also insbesondere Eisen, Nickel-Eisen, Kobalt-Eisen, Silizium-Eisen oder Mu-Metall. Hierbei ist für den Stator 4 eine Ausgestaltung als Statorblechpaket bevorzugt, bei welcher die Spulenkerne 45 und der Rückschluss 42 geblecht ausgestaltet sind, das heisst sie bestehen aus mehreren dünnen Blechelementen, die gestapelt sind.

Jeder Spulenkern 45 hat also eine L-förmige Ausgestaltung, wobei die Längsschenkel 46 jeweils den sich in axialer Richtung A erstreckenden langen Schenkel des L bilden, und die sich senkrecht zu den Längsschenkeln 46 in radialer Richtung auf den magnetisch wirksamen Kern 3 hin gerichtet erstreckenden Querschenkel 47 jeweils den kurzen Schenkel des L bilden. Die Mehrzahl der stabförmigen Längsschenkel 46, die sich in axialer Richtung A erstrecken und an die Säulen eines Tempels erinnern, hat dem Tempelmotor seinen Namen gegeben.

Jeder Querschenkel 47 bildet jeweils einen Statorpol 41. Die Statorpole 41 sind um den magnetisch wirksamen Kern 3 herum angeordnet, das heisst, im Betriebszustand ist der magnetisch wirksame Kern 3 des Flügelrads 10 von den Statorpolen 41 umgeben. Die Wicklungen 6, die vorzugsweise als konzentrierte Wicklungen 6 ausgestaltet sind, sind an den Längsschenkeln 46 angeordnet. An jedem Längsschenkel 46 ist eine der konzentrierten Wicklungen 6 angeordnet, welche den jeweiligen Längsschenkel 46 umgibt.

In diesem Sinne ist im Rahmen der vorliegenden Anmeldung der Begriff Tempelmotor zu verstehen.

Die Spulenkerne 45 sind äquidistant auf einer Kreislinie angeordnet, sodass die Stirnflächen der Querschenkel 47 den magnetisch wirksamen Kern 3 des Flügelrads umgeben. Der magnetisch wirksame Kern 3 ist als permanentmagnetische Scheibe oder als permanentmagnetischer Ring ausgestaltet, der vorzugsweise diametral magnetisiert ist. Die Magnetisierung des magnetisch wirksamen Kerns 3 ist in Fig. 5 und in Fig. 6 jeweils durch den Pfeil ohne Bezugszeichen angedeutet.

Der Stator 4 des elektromagnetischen Drehantriebs ist als Lager- und Antriebsstator ausgestaltet, mit welchem das Flügelrad 10 im Betriebszustand berührungslos magnetisch bezüglich des Stators 4 lagerbar ist.

Zudem ist das Flügelrad - wie bereits erwähnt - mittels des Stators 4 berührungslos magnetisch zur Rotation um die Solldrehachse D antreibbar. Die Solldrehachse D bezeichnet dabei diejenige Achse, um welche sich der magnetisch wirksame Kern 3 im Betriebszustand dreht, wenn sich der magnetisch wirksame Kern 3 bezüglich des Stators 4 in einer zentrierten und unverkippten Lage befindet. Diese Solldrehachse D definiert die axiale Richtung A. Üblicherweise stimmt die die axiale Richtung A festlegende Solldrehachse D mit der Mittelachse des Stators 4 überein.

Vorzugsweise ist der elektromagnetische Drehantrieb nach dem Prinzip des lagerlosen Motors ausgestaltet und wird nach diesem Prinzip betrieben. Mit dem Begriff 'lagerloser Motor' ist dabei ein elektromagnetischer Drehantrieb gemeint, bei welchem der magnetisch wirksame Kern 3 - und damit das Flügelrad 10 - vollkommen magnetisch bezüglich des Stators 4 gelagert ist, wobei keine separaten magnetischen Lager vorgesehen sind. Der Stator 4 ist dazu als Lager- und Antriebsstator ausgestaltet, der sowohl Stator 4 des elektrischen Antriebs als auch Stator 4 der magnetischen Lagerung ist. Mit den elektrischen Wicklungen 6 des Stators 4 lässt sich ein magnetisches Drehfeld erzeugen, welches zum einen ein Drehmoment auf das Flügelrad 10 ausübt, das dessen Rotation um die Solldrehachse D bewirkt, und welches zum anderen eine beliebig einstellbare Querkraft in einer zur axialen Richtung A senkrechten radialen Ebene R (Fig. 6) auf den magnetisch wirksamen Kern 3 ausübt, sodass dessen radiale Position aktiv steuerbar bzw. regelbar ist. Die radiale Ebene R definiert die x-y -Ebene eines kartesischen Koordinatensystems, dessen z-Achse in axialer Richtung A verläuft.

Somit sind drei Freiheitsgrade des Flügelrads 10 aktiv regelbar, nämlich seine Rotation sowie seine radiale Position (zwei Freiheitsgrade). Bezüglich dreier weiterer Freiheitsgrade, nämlich seiner Position in axialer Richtung A und Verkippungen bezüglich der zur Solldrehachse D senkrechten radialen Ebene R (zwei Freiheitsgrade), ist das Flügelrad 10 passiv magnetisch, das heisst nicht ansteuerbar, durch Reluktanzkräfte gelagert bzw. stabilisiert. Das Nichtvorhandensein eines separaten magnetischen Lagers bei vollständiger magnetischer Lagerung des magnetisch wirksamen Kerns 3 ist die Eigenschaft, welcher der lagerlose Motor seinen Namen verdankt. In dem Lager- und Antriebsstator 4 lässt sich die Lagerfunktion nicht von der Antriebsfunktion separieren.

Beim lagerlosen Motor werden im Unterschied zu klassischen Magnetlagern die magnetische Lagerung und der Antrieb des Motors über elektromagnetische Drehfelder realisiert. Typischerweise wird in dem lagerlosen Motor die magnetische Antriebs- und Lagerfunktion durch die Überlagerung zweier magnetischer Drehfelder generiert, die üblicherweise als Antriebs- und Steuerfeld bezeichnet werden. Diese beiden mit den Wicklungen 6 des Stators 4 erzeugten Drehfelder haben in der Regel eine Polpaarzahl, die sich um eins unterscheidet. Hat also beispielsweise das Antriebsfeld die Polpaarzahl p, so hat das Steuerfeld die Polpaarzahl p+1 oder p-1. Dabei werden mit dem Antriebsfeld auf den magnetisch wirksamen Kern 3 in der radialen Ebene wirkende Tangentialkräfte erzeugt, die ein Drehmoment bewirken, was die Rotation um die axiale Richtung A bewirkt. Durch die Überlagerung des Antriebsfelds und des Steuerfelds lässt sich zusätzlich eine beliebig einstellbare Querkraft auf den magnetisch wirksame Kern 3 in der radialen Ebene erzeugen, mit welcher die Position des magnetisch wirksamen Kerns 3 in der radialen Ebene regelbar ist. Es ist also nicht möglich, den elektromagnetischen Fluss, der von den konzentrierten Wicklungen 6 generiert wird, aufzuteilen in einen (elektro-) magnetischen Fluss, der nur für den Antrieb der Rotation sorgt und einen (elektro-) magnetischen Fluss, der nur die magnetische Lagerung realisiert.

Zur Generierung des Antriebs- und des Steuerfelds ist es einerseits möglich, zwei unterschiedliche Wicklungssysteme zu verwenden, nämlich eines zur Erzeugung des Antriebsfelds und eines zur Erzeugung des Steuerfelds. Die Spulen zur Erzeugung des Antriebsfelds werden dann üblicherweise als Antriebsspulen bezeichnet und die Spulen zur Erzeugung des Steuerfelds als Steuerspulen. Der Strom, der in diese Spulen eingeprägt wird, wird dann als Antriebsstrom bzw. als Steuerstrom bezeichnet. Andererseits ist es aber auch möglich, die Antriebs- und Lagerfunktion mit nur einem einzigen Wicklungssystem wie bei der hier beschriebenen Ausführungsform zu generieren, sodass es also keine Unterscheidung zwischen Antriebs- und Steuerspulen gibt. Dies kann so realisiert werden, dass die von einer Kontrolleinrichtung (nicht dargestellt) jeweils ermittelten Werte für den Antriebs- und den Steuerstrom rechnerisch -also z. B. mit Hilfe von Software - addiert bzw. überlagert werden und der sich daraus ergebende Gesamtstrom in die jeweilige konzentrierte Wicklung 6 eingeprägt wird. In diesem Fall ist es natürlich nicht mehr möglich, zwischen Steuer- und Antriebsspulen zu unterscheiden.

Bei der hier beschriebenen Ausführungsform des Stators 4 ist also um jeden Längsschenkel 46 herum jeweils genau eine konzentrierte Wicklung 6 angeordnet. Mit diesen konzentrierten Wicklungen 6 werden im Betriebszustand diejenigen elektromagnetischen Drehfelder erzeugt, mit welchen ein Drehmoment auf Flügelrad 10 bewirkt wird, und mit welchen eine beliebig einstellbare Querkraft in radialer Richtung auf den magnetisch wirksamen Kern 3 des Flügelrads 10 ausübbar ist, sodass die radiale Position des magnetisch wirksamen Kerns 3, also seine Position in der zur axialen Richtung A senkrechten radialen Ebene R, aktiv steuerbar bzw. regelbar ist.

Die Kontrolleinrichtung, welche beispielsweise die Leistungselektronik für die Ansteuerung und die Versorgung der Wicklungen 6 sowie
Signalverarbeitungselektronik umfasst, ist vorzugsweise in dem Statorgehäuse 5 angeordnet.

Weitere Details zum Aufbau und zur Ausgestaltung des lagerlosen Motors finden sich beispielsweise in der EP 4 083 431.

Das Statorgehäuse 5 bildet vorzugsweise eine Ummantelung des Stators 4. Der Stator 4 ist vorzugsweise durch das Statorgehäuse 5 gekapselt. Vorzugsweise ist die Kapselung des Stators 4 als hermetische Kapselung ausgestaltet. Das Statorgehäuse 5 besteht vorzugsweise aus Materialien, die elektrisch nicht leitfähig oder zumindest nur schwach leitfähig und trotzdem strapazierfähig sind. Zudem ist das Statorgehäuse 5 aus Materialien gefertigt, die lösungsmittelbeständig sind, so dass das Statorgehäuse 5 mit Alkohol oder anderen Lösungsmitteln gereinigt werden kann. Zu den nicht leitfähigen Materialien gehören beispielsweise Kunststoffe wie Polyetheretherketon (PEEK) oder Polyoxymethylen Copolymer (POM-C). Zu den relativ schlecht leitfähigen Materialien gehören Metalllegierungen wie beispielsweise Hastelloy oder hochlegierte Titan Legierungen (beispielsweise Grade 5).

Ferner ist es eine bevorzugte Massnahme, dass das Statorgehäuse 5 mit den darin angeordneten Komponenten des Stators 4 vorzugsweise mit einem Kunststoff ausgegossen wird, beispielsweise mit einem Epoxidharz, Polyuretan oder Silikon. Das hat den Vorteil, dass der Stator 4 sehr formstabil bleibt, selbst wenn das Statorgehäuse 5 dünnwandig ausgestaltet ist.

Fig. 7 zeigt in einer sehr schematischen Schnittdarstellung eine Ausführungsform des Flügelrads 10 in einem Schnitt in axialer Richtung A. Das Flügelrad 10 umfasst das Rotorgehäuse 2, in welchem der magnetisch wirksame Kern 3 fixiert ist, sowie die Flügel 7, die auf der oberen Stirnfläche 25 des Rotorgehäuses 2 angeordnet und drehfest mit dem Rotorgehäuse 2 verbunden sind.

Das Rotorgehäuse 2 umfasst die Umkapselung 21 oder ist als Umkapselung 21 ausgestaltet, mittels welcher der magnetisch wirksame Kern 3 gekapselt ist. Die Umkapselung 21, welche den magnetisch wirksamen Kern 3 vollständig umschliesst, besteht vorzugsweise aus einem Kunststoff. Es ist beispielsweise möglich, den magnetisch wirksamen Kern 3 in einen Kunststoff einzugiessen. Auch ist es möglich, das Rotorgehäuse 2 zweiteilig auszugestalten, nämlich mit einem becherförmigen Grundteil und einem darauf angeordneten Deckel (nicht dargestellt). Der magnetisch wirksame Kern 3 wird dann in das Grundteil eingelegt und dort fixiert. Anschliessend wird der Deckel auf das Grundteil aufgesetzt und fest mit diesem verbunden, beispielsweise durch Verschweissen oder durch Verkleben. Das Fixieren des magnetisch wirksamen Kerns 3 in dem Grundteil kann beispielsweise mittels einer Pressfügung erfolgen, sodass eine Presspassung entsteht. Es ist aber auch möglich, das Grundteil nach dem Einlegen des magnetisch wirksamen Kerns 3 mit einem Kunststoff auszugiessen, um damit den magnetisch wirksamen Kern 3 bezüglich des Rotorgehäuses 2 zu fixieren.

Bei der in Fig 7 dargestellten Ausführungsform ist eine Entlastungsbohrung 23 vorgesehen, die sich in axialer Richtung A vollständig durch das Rotorgehäuse 2 und damit durch das Flügelrad 10 hindurch erstreckt. Die Entlastungsbohrung 23 ist zentral im Rotorgehäuse 2 angeordnet und erstreckt sich vom Zentrum der oberen Stirnfläche 25 bis zum Zentrum der unteren Stirnfläche 26. Die Entlastungsbohrung 23 kann beispielsweise - wir in Fig. 7 dargestellt - als zylindrische Bohrung ausgestaltet sein. Es ist aber auch möglich, die Entlastungsbohrung konisch auszugestalten oder eine Mehrzahl von Entlastungsbohrungen vorzusehen, die alle parallel zueinander verlaufen, und die um das Zentrum der oberen Stirnseite 25 bzw. der unteren Stirnseite 26 herum angeordnet sind, wobei die Anordnung vorzugsweise symmetrisch bezüglich der Zentren der Stirnflächen 25, 26 ist.

Wie dies an sich bekannt ist, dient/dienen die Entlastungsbohrung(en) 23 dazu, den durch die rotierenden Flügel 7 generierten Axialschub zumindest teilweise auszugleichen, wodurch die axiale Lagerung des Flügelrads 10 entlastet wird.

Bei der in Fig. 7 dargestellten Ausführungsform ist der magnetisch wirksame Kern 3 ringförmig ausgestaltet und erstreckt sich um die Entlastungsbohrung 23 herum. Der magnetisch wirksame Kern kann als Permanentmagnet ausgestaltet sein oder einen oder mehrerer Permanentmagnete umfassen. Auch ist es möglich, den magnetisch wirksamen Kern als Reluktanzläufer, also ohne Permanentmagnete auszugestalten.

Jeder Flügel 7 erstreckt sich in allgemein radialer Richtung von einem radial innenliegenden Beginn 75 bis zu einem radial aussenliegenden Ende 76, welches bezüglich der radialen Richtung über das Rotorgehäuse 2 hinausragt. Jeder Flügel 7 ist mit einem Radialabstand D1 von der Solldrehachse D angeordnet, das heisst der radial innenliegende Beginn 75 jedes Flügels 7 hat den von null verschiedenen Radialabstand D1 von der Solldrehachse D. Vorzugsweise ist der Radialabstand D1 für alle Flügel 7 identisch.

Jeder Flügel 7 hat jeweils eine Vorderseite 71, eine Rückseite 72, eine Oberkante 73 und eine Unterkante 74.

Die Vorderseite 71 und die Rückseite 72 sind die Flächen des Flügels 7 die primär für das Mischen der Substanzen verantwortlich sind. Die Vorderseite 71 eilt beim Rotieren des Flügelrads 10 der Rückseite 72 voraus. Die Rotation des Flügelrads ist in Fig. 7 durch die Pfeile mit dem Bezugszeichen U angedeutet. Die Vorderseite 71 ist also diejenige Seite des Flügels 7, die manchmal auch als Druckseite bezeichnet wird, während die Rückseite 72 diejenige Seite des Flügels 7 ist, die manchmal auch als Saugseite bezeichnet wird.

Die Oberkante 73 und die Unterkante 74 begrenzen den Flügel 7 bezüglich der axialen Richtung A, wobei die Oberkante 73 an dem Ende des Flügels 7 angeordnet ist, welches dem Rotorgehäuse 2 abgewandt ist, und wobei die Unterkante 74 diejenige Kante ist, die auf oder in dem Rotorgehäuse 2 angeordnet ist.

An jedem Flügel 7 ist ferner ein Stabilisierungselement 9 zur Stabilisierung des Flügelrads 7 gegen Verkippungen vorgesehen. Auf die Anordnung und Ausgestaltung der Stabilisierungselemente 9 wird weiter hinten noch detailliert eingegangen.

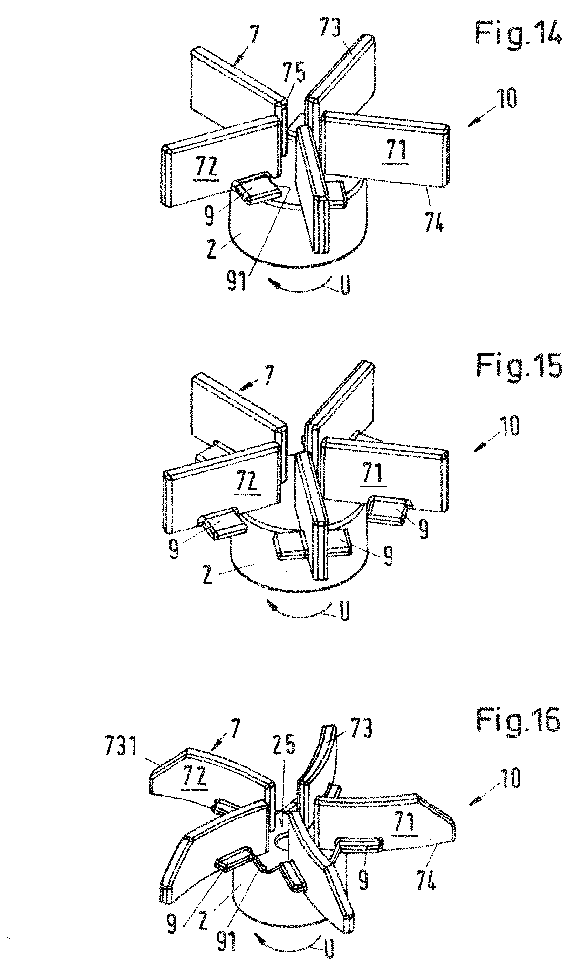

Jedes Stabilisierungselement 9 ist auf der Vorderseite 71 oder auf der Rückseite 72 eines Flügels 7 angeordnet. Natürlich kann auch auf jeder Vorderseite 71 und auf jeder Rückseite 72 jeweils ein Stabilisierungselement 9 angeordnet sein (siehe z.B. Fig. 15).

Jedes Stabilisierungselement 9 erstreckt sich bezüglich der radialen Richtung jeweils von einer radialen Innenkante 91 bis zu einer radialen Aussenkante 92 des Stabilisierungselements 9. Dabei ist jedes Stabilisierungselement 9 so ausgestaltet und angeordnet, dass jede radiale Aussenkante 92 einen Abstand D92 von der Solldrehachse D hat, der kleiner ist als der halbe Aussendurchmesser DA des Flügelrads 10. Das bedeutet, dass die Stabilisierungselemente 9 nicht an dem radial aussenliegenden Ende 76 des jeweiligen Flügels 7 angeordnet sind, sondern von diesem bezüglich der radialen Richtung beabstandet sind. Bezüglich der radialen Richtung enden die Stabilisierungselemente 9 also weiter innen als die Flügel 7.

Es sind Ausgestaltungen möglich, bei denen jede radiale Aussenkante 92 einen Abstand D92 von der Solldrehachse D hat, der höchstens 90% oder höchstens 80% oder höchstens 70% des halben Aussendurchmessers AD des Flügelrads 10 beträgt.

Auch sind Ausgestaltungen möglich, bei denen jede radiale Aussenkante 92 einen Abstand D92 von der Solldrehachse D hat, der höchstens die Hälfte, das heisst höchstens 50%, des halben Aussendurchmessers AD des Flügelrads 10 beträgt siehe z.B. Fig. 14)

Ferner ist es bevorzugt, dass die radiale Innenkante 91 jedes Stabilisierungselements 9 einen Abstand D91 von der Solldrehachse D hat, der grösser ist als der Radialabstand D1 der Flügel 7 von der Solldrehachse D. Das heisst bezüglich der radialen Richtung liegen die radialen Innenkanten 91 der Stabilisierungselements 9 weiter aussen als der radial innenliegende Beginn 75 der Flügel 7.

Fig. 8 zeigt in einer perspektivischen Schnittdarstellung eine Ausgestaltung, bei welcher auf das Rotorgehäuse 2 eine Nabenscheibe 78 aufgesetzt ist. Die Flügel 7 und der magnetisch wirksame Kern 3 sind in Fig. 8 nicht dargestellt. Bei der perspektivischen Darstellung der Fig. 8 ist zum besseren Verständnis ein Segment aus dem Rotorgehäuse 2 und aus der Nabenscheibe 78 herausgeschnitten.

Der magnetisch wirksame Kern 3 (in Fig. 8 nicht dargestellt) ist in dem Rotorgehäuse 2 eingekapselt, vorzugsweise hermetisch eingekapselt. Die Nabenscheibe 78 ist auf der oberen Stirnfläche 25 des Rotorgehäuses 2 angeordnet und hat einen Durchmesser, welcher zumindest ungefähr gleich gross ist wie der Gehäusedurchmesser DR des Rotorgehäuses 2 oder der Durchmesser der oberen Stirnfläche 25.

Wie dies in Fig. 8 zu erkennen ist, ist die obere Stirnfläche 25 mit einer Fase 251 ausgestaltet, die sich entlang des gesamten Umfangs der Stirnfläche 25 erstreckt, das heisst der Übergangsbereich von der oberen Stirnfläche 25 in die Mantelfläche des Rotorgehäuses 2 ist abgeschrägt ausgestaltet.

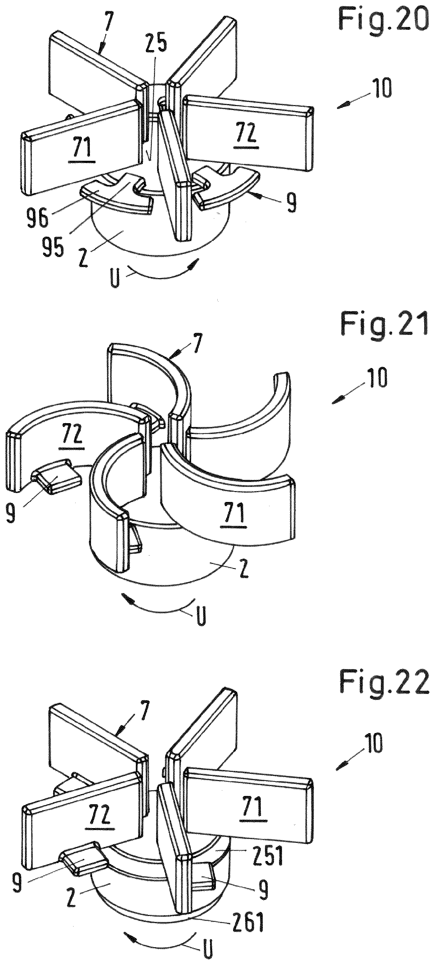

Alternativ oder ergänzend ist die untere Stirnfläche 26 mit einer Fase 261 (in Fig. 8 nicht dargestellt, siehe aber z.B. Fig. 22) ausgestaltet, die sich entlang des gesamten Umfangs der unteren Stirnfläche 26 erstreckt.

Besonders bevorzugt sind sowohl die obere Stirnfläche 25 als auch die untere Stirnfläche 26 des Rotorgehäuses 2 jeweils mit einer Fase 251, 261 ausgestaltet. Durch die Fasen 251, 261 vergrössert sich der Verkippungsspielraum, den das Flügelrad 10 hat. Mit den Fasen 251, 261 kann das Flügelrad 10 weiter verkippt werden, ohne dass das Rotorgehäuse 2 in körperlichen Kontakt mit dem Becher 81 kommt.

Die Nabenscheibe 78 ist fest mit dem Rotorgehäuse 2 verbunden. Wie dies in Fig. 8 dargestellt ist, kann die Nabenscheibe 78 über eine Rastverbindung 27 mit der oberen Stirnfläche 25 des Rotorgehäuses 2 verbunden werden, sodass die Nabenscheibe 78 auf der oberen Stirnfläche 25 einrastbar ist. Solche Einrastmechanismen sind dem Fachmann in zahlreichen Ausgestaltungen bekannt und bedürfen hier keiner näheren Erläuterung. Vorzugsweise wird der Einrastmechanismus so ausgestaltet, dass die Kräfte welche das Drehmoment auf das Flügelrad 10 generieren, den Einrastmechanismus nicht belasten.

Natürlich ist es auch möglich, die Nabenscheibe 78 mittels einer anderen Verbindung auf der oberen Stirnfläche 25 zu befestigen, beispielsweise mittels einer Bajonettverbindung oder einer Schnappverbindung oder mit einer unlösbaren Verbindung wie z.B. eine Verschweissung oder eine Verklebung.

In der Nabenscheibe 78 sind mehrere Aufnahmen 781 vorgesehen, in welche die Flügel 7 eingesetzt werden können. Die Aufnahmen 781 können derart ausgestaltet sein, dass sie gleichzeitig eine Fixierung des jeweiligen Flügels 7 relativ zur Nabenscheibe 78 bewirken. Natürlich ist es auch möglich, die Flügel 7 mittels anderer Methoden in den Aufnahmen 781 zu fixieren, beispielsweise mittels Klebens oder Schweissens.

Ferner sind Ausgestaltungen möglich, bei denen die Flügel 7 integraler Bestandteil der Nabenscheibe 78 sind, also einstückig mit der Nabenscheibe 78 ausgestaltet sind.

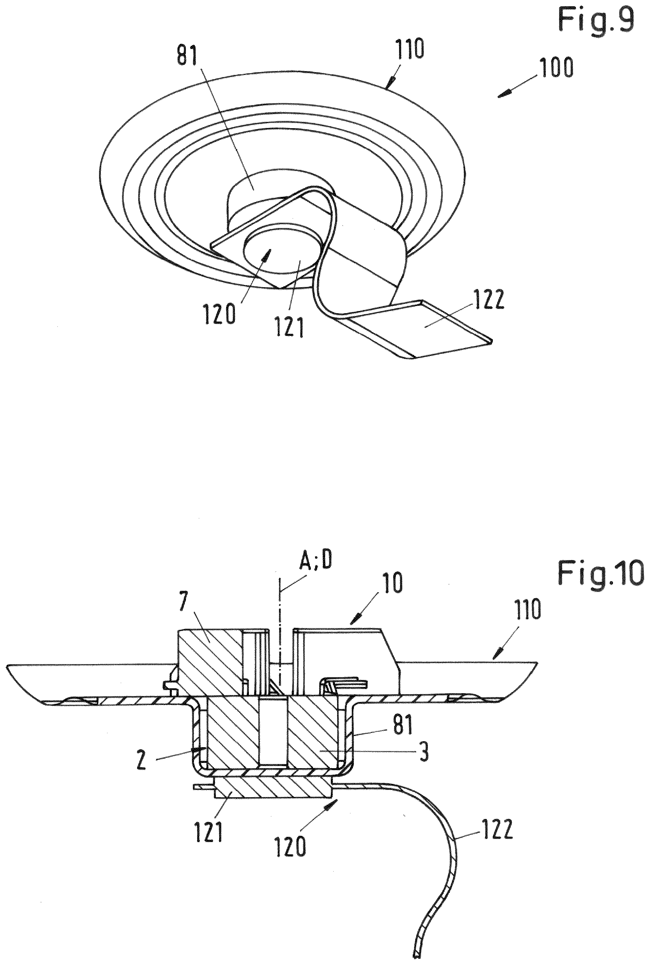

Fig. 9 zeigt in einer perspektivischen Darstellung ein Ausführungsbeispiel der Einmalvorrichtung 100 mit einer Fixiereinrichtung 120 für das Flügelrad 10, falls dieses noch nicht in den Stator 4 eingesetzt ist, beispielsweise während des Transports der Einmalvorrichtung 100. Zum besseren Verständnis zeigt Fig. 10 diese Einmalvorrichtung 100 noch in einem Schnitt entlang der axialen Richtung A.

Die Einmalvorrichtung 100 umfasst das Flügelrad 10, den Becher 81 sowie die Fixiereinrichtung 120. Die Fixiereinrichtung 120 ist derart ausgestaltet, dass sie mittels magnetischer Kräfte den magnetisch wirksamen Kern 3 -und damit das Flügelrad 10- in dem Becher 81 hält.

Für die bevorzugte permanentmagnetische Ausgestaltung des magnetisch wirksamen Kerns 3 umfasst die Fixiereinrichtung 120 eine weichmagnetische Scheibe 121, an welcher ein Band 122 befestigt ist.

Das Flügelrad 10 wird in den Becher 81 eingesetzt, sodass das Rotorgehäuse 2 mit dem darin angeordneten magnetisch wirksamen Kern 3 auf dem Boden des Bechers 81 aufliegt. Anschliessend wird die weichmagnetische Scheibe 121 von aussen an dem Boden des Bechers 81 platziert, sodass sich der Boden des Bechers 81 zwischen dem Rotorgehäuse 2 und der weichmagnetischen Scheibe 121 befindet. Aufgrund der magnetischen Wechselwirkung zwischen der weichmagnetischen Scheibe 121 und dem permanentmagnetisch ausgestalteten magnetisch wirksamen Kern 3 wird das Rotorgehäuse 2 - und damit das Flügelrad 10 in dem Becher 81 fixiert.

Bevor der Becher 81 in die Ausnehmung 51 des Statorgehäuses 5 eingesetzt wird, kann die Fixiereinrichtung 120 durch Ziehen an dem Band 122 entfernt werden, wodurch die weichmagnetische Scheibe 121 vom Boden des Bechers 81 getrennt wird.

Die Fixiereinrichtung 120 ist insbesondere für den Transport der Einmalvorrichtung 100 vorteilhaft, weil mit der Fixiereinrichtung 120 das Flügelrad 10 in einer wohldefinierten Position, nämlich in dem Becher 81, gehalten wird.

Falls der magnetisch wirksame Kern 3 als Reluktanzläufer oder ohne einen Permanentmagneten ausgestaltet ist, wird anstelle der weichmagnetischen Scheibe 121 eine permanentmagnetische Scheibe verwendet.

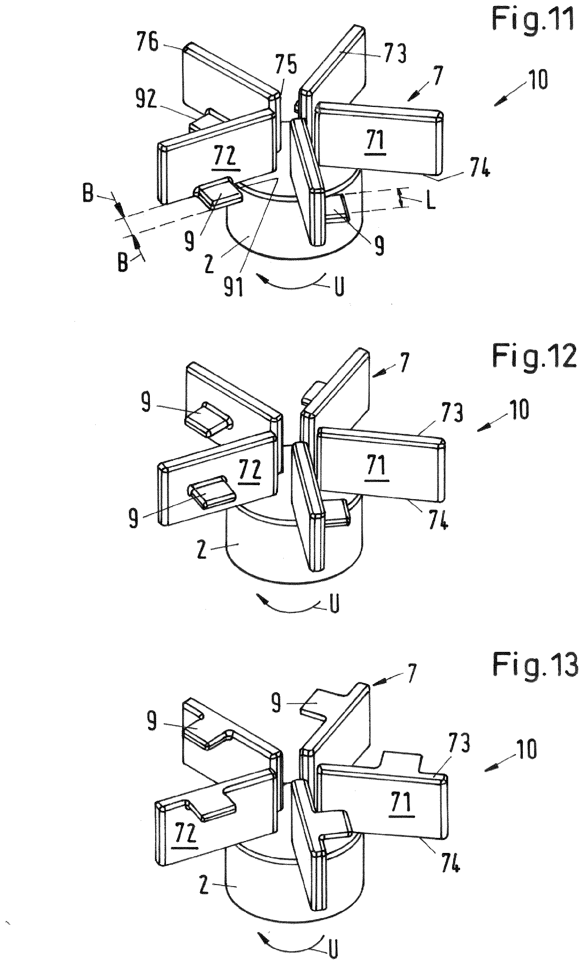

Im Folgenden werden anhand der Fig. 11 - Fig. 24 verschiedene Varianten für die Ausgestaltung des Flügelrads 10 mit den Stabilisierungselementen 9 erläutert. Es versteht sich, dass die vorangehenden Erläuterungen und insbesondere die Erläuterungen bezugnehmend auf die Fig. 7 und die Fig. 8 in gleicher Weise oder in sinngemäss gleicher Weise auch für die im Folgenden beschriebenen Varianten gelten. Ferner sei betont, dass spezifische Massnahmen, die beispielhaft anhand einer der Varianten erläutert sind, auch mit Massnahmen und Ausgestaltungen kombiniert werden können, die anhand andere Varianten erläutert werden, d.h. die jeweils erläuterten Massnahmen und Ausgestaltungen können auch mit anderen Varianten kombiniert werden.

Auch versteht es sich, dass jede der Varianten auch mit einer Nabenscheibe ausgestaltet sein kann.

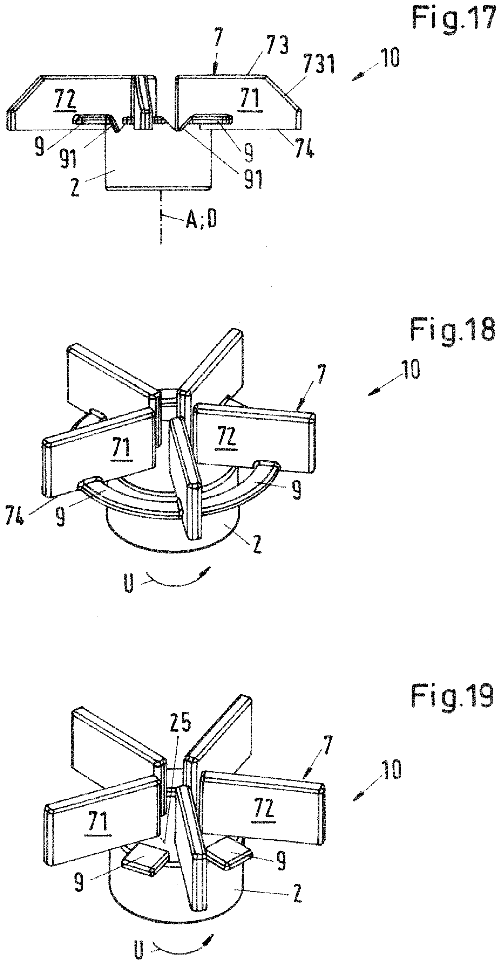

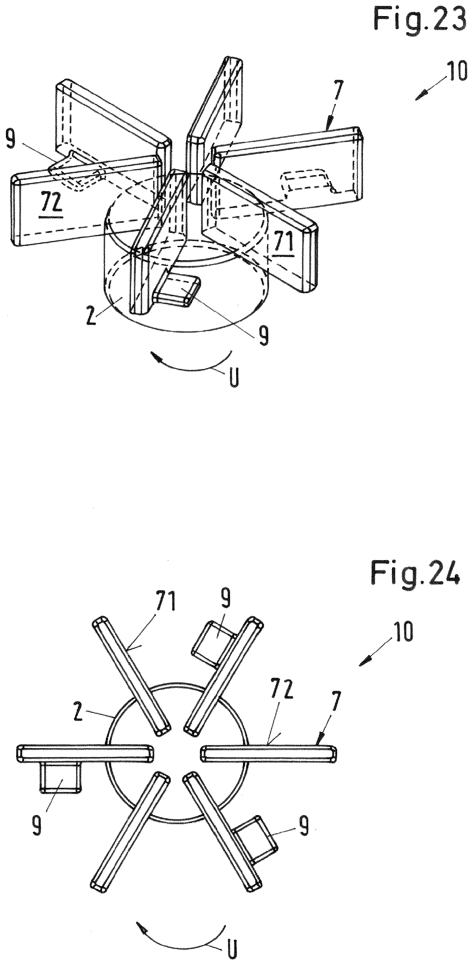

Mit Ausnahme von Fig. 17 und Fig. 24 zeigen alle Fig. 11-24 jeweils eine Variante für die Ausgestaltung des Flügelrads 10 in einer perspektivischen Darstellung. Fig. 17 zeigt die Variante aus Fig. 16 in einer Seitenansicht. Fig. 24 zeigt die Variante aus Fig. 23 in einer Aufsicht aus der axialen Richtung.

Bei allen im Folgenden beschriebenen Varianten ist das Flügelrad 10 jeweils mit genau fünf Flügeln 7 ausgestaltet, die äquidistant entlang des Umfangs des Flügelrads 10 angeordnet sind. Auch wenn die Ausgestaltung mit genau fünf Flügeln 7 eine bevorzugte Ausgestaltung ist, so ist die Erfindung natürlich nicht auf eine Anzahl von genau fünf Flügeln beschränkt. Das Flügelrad 10 kann auch mit weniger als fünf Flügeln 7, beispielsweise mit genau drei oder genau vier Flügeln 7 ausgestaltet sein, oder mit mehr als fünf Flügeln 7, beispielsweise mit genau sechs oder genau sieben Flügeln 7.

Erfindungsgemäss sind die Stabilisierungselemente 9 zur Stabilisierung des Flügelrads 10 gegen Verkippungen jeweils zwischen zwei Flügeln 7 angeordnet. Dieses Merkmal umfasst die beiden grundsätzlichen Möglichkeiten, dass die Stabilisierungselemente 9 direkt am Rotorgehäuse 2 bzw., falls vorhanden, an der Nabenscheibe 78, und bezüglich der Umfangsrichtung zwischen zwei Flügeln 7 angeordnet sind, oder dass die Stabilisierungselements 9 jeweils an der Vorderseite 71 oder an der Rückseite 72 eines Flügels 7 angeordnet sind. Auch bei der Anordnung an der Vorderseite 71 oder an der Rückseite 72 eines Flügels 7 ist dieses Stabilisierungselement 9 zwischen zwei Flügeln 7 angeordnet, nämlich zwischen dem Flügel 7, an dessen Vorderseite 71 oder Rückseite 72 das Stabilisierungselement 9 angeordnet ist, und dem in Umfangsrichtung gesehen benachbarten Flügel 7. Auch sind Kombinationen dieser beiden Möglichkeiten realisierbar, nämlich Ausgestaltungen des Flügelrads 10, bei denen sowohl direkt am Rotorgehäuse 2 bzw. an der Nabenscheibe 78 als auch an den Vorderseiten 71 bzw. Rückseiten 72 der Flügel 7 Stabilisierungselemente 9 angeordnet sind.

Fig. 11 zeigt eine Variante, bei welcher an der Rückseite 72 jedes Flügels 7 jeweils ein Stabilisierungselement 9 vorgesehen ist. Jedes Stabilisierungselement 9 ist als rechteckige Platte ausgestaltet, welche eine Länge L und eine Breite B aufweist. Dabei ist die Länge L die Erstreckung des Stabilisierungselements 9 in radialer Richtung und die Breite B ist die Erstreckung in der dazu und zur axialen Richtung A senkrechten Richtung. Die Breite B gibt also an, wie weit sich das Stabilisierungselement 9 in den Zwischenraum zwischen zwei benachbarten Flügeln 7 erstreckt. Alle Stabilisierungselemente 9 sind zumindest im Wesentlichen gleich ausgestaltet. Die Länge L jedes Stabilisierungselements 9 ist deutlich kleiner als die entsprechende Länge des Flügels 7, wobei die Länge des Flügels 7 die entlang der Vorderseite 71 (oder der Rückseite 72) gemessene Entfernung des radial aussenliegenden Endes 76 vom radial innenliegenden Beginn 75 des Flügels 7 ist. Vorzugsweise ist die Länge L des Stabilisierungselements 9 höchstens die Hälfte oder höchstens ein Drittel der Länge des Flügels 7.

Bezüglich der axialen Richtung A sind die Stabilisierungselemente 9 jeweils an der Unterkante 74 des Flügels 7 angeordnet. Bezüglich der radialen Richtung sind die Stabilisierungselemente 9 etwa mittig am Flügel 7 angeordnet, sodass der Abstand der radialen Innenkante 91 des Stabilisierungselements 9 vom radial innenliegenden Beginn 75 des Flügels 7 in etwa gleich gross ist wie der Abstand der radialen Aussenkante 92 des Stabilisierungselements 9 von dem radial aussenliegenden Ende 76 des Flügels 7.

Natürlich ist es auch möglich, dass die Stabilisierungselemente 9 jeweils an der Vorderseite 71 eines Flügels 7 angeordnet sind.

Bei der in Fig. 11 dargestellten Variante sind die plattenförmigen Stabilisierungselemente 9 jeweils rechtwinklig zu der Rückseite 72 des Flügels 7 angeordnet, das heisst jedes Stabilisierungselement 9 schliesst mit der Rückseite 72, an der es angeordnet ist, jeweils einen Winkel von 90° ein. In anderen Ausgestaltungen ist jedes Stabilisierungselement 9 schräg zur Rückseite 72 (oder zur Vorderseite 71) angeordnet, sodass das Stabilisierungselement 9 mit der jeweiligen Rückseite 72 (oder Vorderseite 71) einen stumpfen Winkel oder eine spitzen Winkel einschliesst. Bevorzugt ist ein Winkel der im Bereich von 40° bis 140° liegt.

Fig. 12 zeigt eine Variante, bei welcher die Stabilisierungselemente 9 bezüglich der axialen Richtung A etwa mittig zwischen der Oberkante 73 und der Unterkante 74 des jeweiligen Flügels 7 angeordnet sind.

Fig. 13 zeigt eine Variante, bei welcher die Stabilisierungselemente 9 bezüglich der axialen Richtung A an der Oberkante 73 des jeweiligen Flügels 7 angeordnet sind.

Fig. 14 zeigt eine Variante, bei welcher die Stabilisierungselemente 9 bezüglich der axialen Richtung A wiederum an der Unterkante 74 des jeweiligen Flügels 7 angeordnet sind, aber im Vergleich zu der in Fig. 11 gezeigten Variante bezüglich der radialen Richtung weiter innen, also näher an der Solldrehachse D, angeordnet sind. Jedes Stabilisierungselement 9 ist so angeordnet, dass seine radiale Innenkante 91 an oder in der Nähe des radial innenliegenden Beginns 75 des jeweiligen Flügels 7 angeordnet ist.

Fig. 15 zeigt eine Variante, bei welcher die Stabilisierungselemente 9 symmetrisch angeordnet sind, derart, dass jeder Flügel 7 sowohl an seiner Vorderseite 71 als auch an seiner Rückseite 72 jeweils ein Stabilisierungselement 9 aufweist, wobei die am selben Flügel 7 vorgesehenen Stabilisierungselemente 9 symmetrisch bezüglich des Flügels 7 angeordnet sind, also an der gleichen Position bezüglich der axialen Richtung A als auch bezüglich der radialen Richtung.

Dabei sind natürlich alle Positionen bezüglich der axialen Richtung A und bezüglich der radialen Richtung möglich, insbesondere die in den Fig. 11 - Fig. 14 dargestellten Positionen.

Die symmetrische Anordnung ist eine besonders bevorzugte Massnahme.

Die Fig. 16 und Fig. 17 zeigen eine Variante, welche den zusätzlichen Vorteil hat, dass die mechanische Festigkeit der Flügel 7 durch die Stabilisierungselemente 9 deutlich erhöht wird. Fig. 16 zeigt die Variante in einer perspektivischen Darstellung und Fig. 17 zeigt die Variante in einer Seitenansicht. Bei dieser Variante sind die Stabilisierungselemente 9 bezüglich ihrer Position in axialer Richtung A jeweils in der Nähe der Unterkante 74 des Flügels angeordnet, jedenfalls deutlich näher an der Unterkante 74 als an der Oberkante 73.

Die radialen Innenkanten 91 der Stabilisierungselemente 9 sind dabei so ausgestaltet, dass sie jeweils bis auf die obere Stirnfläche 25 des Rotorgehäuses 2 gezogen sind, sodass jedes Stabilisierungselement 9 nicht nur mit dem jeweiligen Flügel 7 sondern auch mit dem Rotorgehäuse 2 körperlich verbunden ist. Die daraus resultierende Erhöhung der mechanischen Festigkeit der Flügel 7 ermöglicht grössere und insbesondere längere Flügel 7. Hierdurch kann die Mischvorrichtung 1 bei gleicher Mischeffizienz mit langsamer drehenden Flügeln 7 betrieben werden, womit sich der Beginn unerwünschter Kavitationen zu höheren Drehmomenten verschiebt.

Als eine weitere Massnahme sind die Flügel 7 in radialer Richtung gesehen gekrümmt ausgestaltet, sodass die Vorderseite 71 der Flügel jeweils konvex ausgestaltet ist und die Rückseite 72 jeweils konkav.

Als eine weitere Option sind die Oberkanten 73 der Flügel 7 in ihrem radial äusseren Bereich mit einer Abschrägung 731 versehen, sodass die Erstreckung des Flügels 7 in axialer Richtung A in diesem radial äusseren Bereich mit zunehmendem Abstand von der Solldrehachse D abnimmt. Die Abschrägung 731 kann geradlinig oder gekrümmt ausgestaltet sein.

Weiter Möglichkeiten der Ausgestaltung bestehen darin, dass auch die Unterkante 74 der Flügel 7 in ihrem radial äusseren Bereich mit einer Abschrägung ausgestaltet ist, oder dass die Oberkante 73 und/oder die Unterkante 74 jeweils in ihrem radial innenliegenden Bereich mit einer geradlinigen oder einer gekrümmten Abschrägung versehen sind. Auch sind Ausgestaltungen möglich, bei denen jeder Flügel 7 derart ausgestaltet ist, dass er sich mit zunehmendem Abstand von der Solldrehachse D kontinuierlich verjüngt, also schmäler bezüglich der Erstreckung in axialer Richtung A wird.

Fig. 18 zeigt eine Variante bei welcher die Stabilisierungselemente 9 als Ringsegmente ausgestaltet sind, wobei sich jedes Stabilisierungselement 9 jeweils von der Vorderseite 71 eines Flügels 7 bis zu der Rückseite 72 des in Umfangsrichtung benachbarten Flügels 7 erstreckt, und wobei das Stabilisierungselement mit beiden Flügeln 7 fest verbunden ist.

Bei der in Fig. 18 gezeigten Ausgestaltung sind die ringsegmentförmigen Stabilisierungselemente 9 jeweils an oder benachbart zu der Unterkante 74 der Flügel 7 angeordnet. Natürlich sind auch Ausgestaltungen möglich, bei denen die ringsegmentförmigen Stabilisierungselemente 9 an anderen Positionen bezüglich der axialen Richtung A angeordnet sind.

Im Allgemeinen sind solche Ausgestaltungen bevorzugt, bei denen die Stabilisierungselemente 9 bezüglich der axialen Richtung A nahe am magnetisch wirksamen Kern 3 und bezüglich der radialen Richtung weiter innenliegend, also näher an der Solldrehachse D angeordnet sind.

In den Fig. 19 und Fig. 20 sind Varianten dargestellt, bei welchen die Stabilisierungselemente 9 direkt am Rotorgehäuse 2, nämlich auf der oberen Stirnfläche 25 des Rotorgehäuses 2, angeordnet sind. Es versteht sich, dass bei Ausgestaltungen mit der Nabenscheibe 78 die Stabilisierungselemente 9 in sinngemäss gleicher Weise auf der Nabenscheibe 78 angeordnet sind.

Bei der in Fig. 19 dargestellten Variante ist jeweils bezüglich der Umfangsrichtung mittig zwischen zwei benachbarten Flügeln 7 ein Stabilisierungselement 9 auf der oberen Stirnfläche 25 des Rotorgehäuses 2 angeordnet, welches bezüglich der radialen Richtung über das Rotorgehäuse 2 hinausragt. Die Stabilisierungselemente 9 sind hier also nicht unmittelbar mit den Flügeln 7 verbunden. Die Stabilisierungselemente 9 sind beispielsweise als rechteckige Platten mit der Länge L und der Breite B (Fig. 11) ausgestaltet.

Bei der in Fig. 20 dargestellten Ausgestaltung sind die Stabilisierungselemente 9 jeweils T-förmig ausgestaltet, wobei ein Ende des Längsschenkels 95 des T mit der oberen Stirnfläche 25 des Rotorgehäuses 2 verbunden ist, und der Querschenkel 96 des T radial aussenliegend angeordnet ist. Zudem ist der Querschenkel 96 des T bogenförmig gekrümmt ausgestaltet.

Fig. 21 zeigt ein Variante, bei welcher die Flügel 7 wiederum gekrümmt ausgestaltet sind, sodass die Vorderseite des Flügels 71 konvex ausgestaltet ist und die Rückseite 72 konkav. Bei dieser Variante ist nur an der Rückseite 72 jedes Flügels 7 jeweils ein Stabilisierungselement 9 vorgesehen.

Fig. 22 zeigt eine Variante, bei der das Rotorgehäuse 2 sowohl an seiner oberen Stirnfläche 25 als auch an seiner unteren Stirnfläche 26 jeweils mit einer Fase 251, 261 ausgestaltet ist. Diese Ausgestaltung mit den beiden Fasen 251 und 261 ist für alle Ausführungsformen des Laufrads 10 bevorzugt.

Es versteht sich, dass bei denjenigen Ausgestaltungen, bei denen die Stabilisierungselemente 9 unmittelbar an der Vorderseite 71 und/oder an der Rückseite 72 der Flügel 7 angeordnet sind, nicht an jedem Flügel 7 ein Stabilisierungselement 9 vorgesehen sein muss.

Die Fig. 23 und Fig. 24 zeigen eine solche Variante, bei welcher die Stabilisierungselemente 9 direkt an den Flügeln 7 angeordnet sind, aber nicht an jedem Flügel 7 ein Stabilisierungselement 9 vorgesehen ist. Es sind also auch Varianten möglich, bei denen einige der Flügel 7 mit einem Stabilisierungselement 9 ausgestaltet sind und einige Flügel 7 ohne ein Stabilisierungselement 9. Fig. 23 zeigt eine solche Variante in einer perspektivischen Darstellung, und Fig. 24 zeigt die Variante in einer Aufsicht aus der axialen Richtung. Bei dieser Variante hat das Flügelrad 10 genau sechs Flügel 7. In Umfangsrichtung gesehen ist an der Rückseite 72 jedes zweiten Flügels 7 jeweils ein Stabilisierungselement 9 angeordnet. Diejenigen Flügel 7, die jeweils zwischen zwei Flügeln 7 mit Stabilisierungselement 9 angeordnet sind, haben keine Stabilisierungselemente. Folglich haben drei der Flügel 7 ein Stabilisierungselement 9 und die anderen drei Flügel 7 haben kein Stabilisierungselement 9.

Es gibt natürlich noch viele weiter Ausgestaltungsmöglichkeiten, so können beispielsweise die Flügel 7 - bei geradliniger Ausgestaltung - so angeordnet werden, dass sie sich nicht in radialer Richtung erstrecken, sondern gegen die radiale Richtung geneigt angeordnet sind. Auch sind Ausgestaltungen möglich, bei denen die Flügel 7 gegen die axiale Richtung A geneigt angeordnet sind, derart das die Unterkante 74 des Flügels 7 bezüglich der Umfangsrichtung versetzt zur Oberkante 73 des gleichen Flügels 7 angeordnet ist

Für die erfindungsgemässe Mischvorrichtung 1, welche die Einmalvorrichtung 100 und die wiederverwendbare Vorrichtung 200 umfasst, ist es ferner ein wichtiger Aspekt, dass die Einmalvorrichtung 100, für einige Anwendungen sterilisierbar sein muss. Dabei ist es besonders vorteilhaft, wenn die Einmalvorrichtung 100 gamma-sterilisierbar ist. Bei dieser Art der Sterilisierung wird das zu sterilisierende Element mit Gamma-Strahlung beaufschlagt. Der Vorteil der Gamma-Sterilisierung, beispielsweise im Vergleich zur Dampfsterilisierung, liegt insbesondere darin, dass die Sterilisierung auch durch die Verpackung hindurch erfolgen kann. Gerade bei Einmalteilen ist es eine gängige Praxis, dass die Teile nach ihrer Herstellung in die Verpackung gebracht werden und dann noch eine Zeit lagern, bevor sie an den Kunden ausgeliefert werden. Die Sterilisierung erfolgt dann üblicherweise erst kurz vor der Auslieferung an den Kunden oder erst durch den Kunden. In solchen Fällen erfolgt die Sterilisierung durch die Verpackung hindurch, was bei einer Dampfsterilisierung oder anderen Verfahren nicht möglich ist.

Bezüglich der Einmalteilvorrichtung 100 ist es in der Regel nicht notwendig, dass sie mehr als einmal sterilisierbar sein muss. Dies ist insbesondere bei der Gamma-Sterilisierung ein grosser Vorteil, weil die Beaufschlagung mit Gamma-Strahlung bei Kunststoffen zu Degradationen führen kann, sodass eine mehrfache Gamma-Sterilisierung den Kunststoff unbrauchbar machen kann.

Da in der Regel bei Einmalvorrichtungen 100 auf eine Sterilisierung unter hohen Temperaturen und /oder unter hohem (Dampf-) Druck verzichtet werden kann, können kostengünstigere Kunststoffe eingesetzt werden, beispielsweise solche, die keine hohen Temperaturen aushalten, oder die nicht mehrfach hohen Temperatur- und Druckwerten ausgesetzt werden können.

Unter Berücksichtigung all dieser Aspekte ist es daher bevorzugt, für die Herstellung der Einmalvorrichtung 100 solche Kunststoffe zu verwenden, die zumindest einmal gamma-sterilisierbar sind. Die Materialien sollten dabei gammastabil für eine Dosis von mindestens 40 kGy sein, um eine einmalige Gamma-Sterilisierung zu ermöglichen. Bei der Gamma-Sterilisierung sollten zudem keine giftigen Stoffe entstehen. Zudem ist es bevorzugt, wenn alle Materialien, die mit den zu mischenden bzw. den durchmischten Substanzen in Berührung kommen, USP Class VI Standards erfüllen.

Für die Herstellung der Einmalvorrichtung 100 sind beispielsweise die folgenden Kunststoffe geeignet: PolyVinylChlorid (PVC), PolyPropylene (PP), PolyEthylene (PE), Low Density PolyEthylene (LDPE), Ultra Low Density PolyEthylene (ULDPE), High Density PolyEthylene (HDPE), Ethylene Vinyl Acetate (EVA), PolyEthylene Terephthalate (PET), PolyVinyliDene Fluoride (PVDF), Acrylonitrile Butadiene Styrene (ABS), PolyUrethan (PU), PolyAcryl, PolyCarbonate (PC), Polysulfone wie beispielsweise Polysulfon (PSU), Silicone.

## Patentansprüche

1. Mischvorrichtung zum Mischen von mindestens zwei Substanzen mit einer Einmalvorrichtung (100), die für den Einmalgebrauch ausgestaltet ist, und mit einer wiederverwendbaren Vorrichtung (200), die für den Mehrfachgebrauch ausgestaltet ist,
wobei die Einmalvorrichtung (100) ein Flügelrad (10) mit einem Rotorgehäuse (2) und mit mehreren Flügeln (7) zum Mischen der Substanzen, sowie einen Becher (81) zur Aufnahme des Rotorgehäuses (2) umfasst, wobei das Flügelrad (10) zum Rotieren um eine Solldrehachse (D) ausgestaltet ist, welche eine axiale Richtung (A) definiert, wobei das Flügelrad (10) einen Aussendurchmesser (DA) aufweist, welcher die maximale Erstreckung des Flügelrads (10) in einer radialen Richtung senkrecht zur axialen Richtung (A) ist, wobei in dem Rotorgehäuse (2) ein magnetisch wirksamer Kern (3) vorgesehen ist, wobei der Becher (81) formstabil ausgestaltet ist,
wobei die wiederverwendbare Vorrichtung (200) einen Stator (4) umfasst, mit welchem das Flügelrad (10) im Betriebszustand berührungslos magnetisch zur Rotation um die Solldrehachse (D) antreibbar und magnetisch bezüglich des Stators (4) lagerbar ist, wobei der Stator (4) mehrere Wicklungen (6) zur Erzeugung eines elektromagnetischen Drehfelds zum Antreiben und Lagern des Flügelrads (10) umfasst, und wobei der Stator ein Statorgehäuse (5) mit einer Ausnehmung (51) aufweist, welche zur Aufnahme des Bechers (81) mit dem darin angeordneten Rotorgehäuse (2) ausgestaltet ist, sodass der Becher (81) in die Ausnehmung einsetzbar ist,
**dadurch gekennzeichnet, dass** an dem Flügelrad (10) mehrere Stabilisierungselemente (9) zur Stabilisierung des Flügelrads (10) gegen Verkippungen vorgesehen sind, wobei jedes Stabilisierungselement (9) zwischen zwei Flügeln (7) angeordnet ist, und sich bezüglich der radialen Richtung von einer radialen Innenkante (91) bis zu einer radialen Aussenkante (92) erstreckt, und wobei jede radiale Aussenkante (92) einen Abstand (D92) von der Solldrehachse (D) hat, der kleiner ist als der halbe Aussendurchmesser (DA) des Flügelrads (7).

2. Mischvorrichtung nach Anspruch 1, wobei jeder Flügel (7) bezüglich der radialen Richtung mit einem Radialabstand (D1) von der Solldrehachse (S) angeordnet ist, wobei der Radialabstand (D1) von null verschieden ist, und wobei die radiale Innenkante (91) jedes Stabilisierungselements einen Abstand (D91) von der Solldrehachse (D) hat, der grösser ist als der Radialabstand (D1).

3. Mischvorrichtung nach einem der vorangehenden Ansprüche, wobei jeder Flügel (7) eine Vorderseite (71), eine Rückseite (72), eine Oberkante (73) und eine Unterkante (74) aufweist, wobei die Vorderseite (71) beim Rotieren des Flügelrads (10) der Rückseite (72) vorausläuft, wobei die Unterkante (74) und die Oberkante (73) den Flügel (7) bezüglich der axialen Richtung (A) begrenzen, wobei die Oberkante (73) an dem Ende des Flügels (7) angeordnet ist, welches dem Rotorgehäuse (2) abgewandt ist, und wobei die Stabilisierungselemente (9) auf den Vorderseiten (71) und/oder auf den Rückseiten (72) der Flügel angeordnet sind.

4. Mischvorrichtung nach Anspruch 3, wobei die Stabilisierungselemente (9) mit der Vorderseite (71) oder mit der Rückseite (72) jeweils einen von 0° und von 180° verschiedenen Winkel einschliessen, der vorzugsweise im Bereich von 40° bis 140° liegt.

5. Mischvorrichtung nach einem der Ansprüche 3-4, wobei die Stabilisierungselemente (9) symmetrisch angeordnet sind, derart, dass jeder Flügel (7), an dessen Vorderseite (71) ein Stabilisierungselement (9) vorgesehen ist, auch auf der Rückseite (72) mit einem Stabilisierungselement (9) versehen ist.

6. Mischvorrichtung nach einem der Ansprüche 3-5, wobei die Stabilisierungselemente (9) näher an der Unterkante (74) als an der Oberkante (73) des jeweiligen Flügels (7) angeordnet sind.

7. Mischvorrichtung nach einem der Ansprüche 3-6, wobei jeder Flügel (7) gekrümmt ausgestaltet ist, sodass die Vorderseite (71) oder die Rückseite (72) konkav ausgestaltet sind.

8. Mischvorrichtung nach einem der Ansprüche 3-7, wobei die Stabilisierungselemente (9) Ringsegmente umfassen, die sich jeweils von der Vorderseite (71) eines Flügels (7) bis zu der Rückseite (72) eines benachbarten Flügels (7) erstrecken.

9. Mischvorrichtung nach einem der vorangehenden Ansprüche, wobei das Rotorgehäuse (2) zylindrisch mit einem äusseren Gehäusedurchmesser (DR) ausgestaltet ist, sowie mit einer oberen Stirnfläche (25) und mit einer unteren Stirnfläche (26), welche das Rotorgehäuse (2) bezüglich der axialen Richtung begrenzen, und wobei optional auf der oberen Stirnfläche (25) des Rotorgehäuses (2) eine scheibenförmige Nabenscheibe (78) angeordnet ist, auf welcher die Flügel (7) vorgesehen sind.

10. Mischvorrichtung nach Anspruch 9, wobei die Stabilisierungselemente (9) auf der oberen Stirnfläche (25) des Rotorgehäuses (2) oder auf der Nabenscheibe (78) angeordnet sind, und wobei der Abstand jeder radialen Aussenkante (92) von der Solldrehachse (D) grösser ist als der halbe Gehäusedurchmesser (DR) des Rotorgehäuses (2).

11. Mischvorrichtung nach einem der Ansprüche 9-10, wobei die obere Stirnfläche (25) und/oder die untere Stirnfläche (26) des Rotorgehäuses mit einer Fase (251, 261) ausgestaltet sind, die sich entlang des gesamten Umfangs der Stirnfläche (25, 26) erstreckt.

12. Mischvorrichtung nach einem der vorangehenden Ansprüche mit mindestens einer Entlastungsbohrung (23), die sich in axialer Richtung (A) vollständig durch das Flügelrad (10) hindurch erstreckt.

13. Einmalvorrichtung für eine Mischvorrichtung, die nach einem der vorangehenden Ansprüche ausgestaltet ist, wobei die Einmalvorrichtung (100) das Flügelrad (10) sowie den Becher (81) zur Aufnahme des Rotorgehäuses (2) umfasst.

14. Einmalvorrichtung nach Anspruch 13, die ferner eine Fixiereinrichtung (120) umfasst, mit welchem der magnetisch wirksame Kern (3) magnetisch in dem Becher (81) gehalten wird, wobei die Fixiereinrichtung (120) durch Ziehen entfernbar ist.

15. Einmalvorrichtung nach einem der Ansprüche 13-14, die ferner einen flexiblen Mischbehälter (110) für die zu mischenden Substanzen umfasst, wobei der Mischbehälter (110) dichtend mit dem Becher (81) zu einer Gesamtheit verbunden ist, und wobei das Flügelrad (10) in der Gesamtheit angeordnet ist.
